(19) Europäisches Patentamt
European Patent Office
Office européen des brevets

(11) **EP 4 102 507 A1**

(12) **EUROPEAN PATENT APPLICATION**

(43) Date of publication:
**14.12.2022 Bulletin 2022/50**

(21) Application number: **22159146.4**

(22) Date of filing: **28.02.2022**

(51) International Patent Classification (IPC):
**G16B 15/30** (2019.01)      **G16B 40/20** (2019.01)
**G16C 20/30** (2019.01)      **G16C 20/50** (2019.01)
**G16C 20/70** (2019.01)

(52) Cooperative Patent Classification (CPC):
**G16B 15/30; G16B 40/20**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **05.03.2021   JP 2021035648**

(71) Applicant: **Ahead Biocomputing, Co. Ltd
Kanagawa 210-0007 (JP)**

(72) Inventors:
• **Akiyama, Yutaka
Tokyo, 152-8550 (JP)**

• **Ohue, Masahito
Tokyo, 152-8550 (JP)**
• **Yanagisawa, Keisuke
Tokyo, 152-8550 (JP)**
• **Yoshikawa, Yasushi
Tokyo, 152-8550 (JP)**
• **Li, Jianan
Tokyo, 152-8550 (JP)**

(74) Representative: **Berggren Oy
P.O. Box 16
Eteläinen Rautatiekatu 10A
00101 Helsinki (FI)**

(54) **PREDICTION DEVICE, TRAINED MODEL GENERATION DEVICE, PREDICTION METHOD, AND TRAINED MODEL GENERATION METHOD**

(57)      A prediction device extracts each predictive feature vector expressing a feature from a peptide that is a target for biostability prediction. The prediction device generates a predicted value of biostability of the prediction target cyclic peptide by inputting plural predictive feature vectors into a trained model pre-trained to output a predicted value of peptide biostability.

FIG.18

START

RECEIVE PEPTIDE INFORMATION OF BIOSTABILITY PREDICTION TARGET PEPTIDE — S500

GENERATE PLURAL CONFORMATIONS ADOPTABLE BY PEPTIDE — S502

FROM THE PLURAL CONFORMATIONS SELECT A CONFORMATION SATISFYING PRESCRIBED SELECTION CRITERIA — S504

EXECUTE DOCKING CALCULATION BETWEEN PEPTIDE CORRESPONDING TO SELECTED CONFORMATION AND BLOOD PLASMA PROTEIN — S506

PREDICT BIOSTABILITY OF PREDICTION TARGET PEPTIDE — S508

OUTPUT RESULT — S510

END

EP 4 102 507 A1

**Description**

BACKGROUND

Technical Field

The present disclosure relates to a prediction device, a trained model generation device, a prediction method, a trained model generation method, a recording medium recorded with a prediction program, and a recording medium recorded with a trained model generation program.

Related Art

A molecular dynamics simulation is disclosed in Japanese Patent Application Laid-Open (JP-A) No. 2017-037378. This takes, as an initial structure in structural analysis of a biopolymer, a structure of outlier values not included in any cluster for clustering performed on plural structures in multidimensional space having all of the index dimensions included in a dimension set as coordinate axes (i.e. in claim 4).

A protein three dimensional structure prediction program disclosed in International Publication (WO) No. 2003/054743 predicts the three dimensional structure of a protein. A computer executes this protein three dimensional structure prediction program, reads in an amino acid sequence for a protein, and predicts secondary structure information. Next, the computer computes a number of amino acids to form a turn based on the secondary structure information, acquires turn structure information of a turn having a high probability of being present from the computed number of amino acids and the secondary structure information, performs prediction-reproduction of a turn, and predicts a three dimensional structure of the protein.

Moreover, Japanese National-Phase Publication No. 2020-523010 discloses a method for generating, for each patient, a set of likelihoods for a set of neoantigens for the patient by inputting a peptide sequence of each of the sets of neoantigens into a machine-learned presentation model (i.e. in claim 1).

Moreover, Japanese National-Phase Publication No. 2020-519246 discloses a method for generating a set of presentation likelihoods for a set of neoantigens by employing a processor of a computer to input numerical vectors of peptides into a deep learning presentation model (i.e. in claim 1).

Peptide drugs have recently become a focus of attention as a type of middle molecule drugs. However, there are many unclear points regarding the pharmacokinetics of peptides. In particular, the stability of peptides in the body (hereafter simply referred to as biostability) is an important factor when a peptide is applied as a drug. There is accordingly a demand to predict with good accuracy whether a peptide obtained for administering as a drug has a certain degree of biostability.

Biostability is a factor governed by a rate of plasma protein binding (PPB) that expresses a rate of binding between a protein, such as albumin, in blood plasma and a peptide. A major problem in conventional small molecule drug discovery is the suppression of lipophilicity of drugs so that the plasma protein binding is not excessively high. However, in peptide drug discovery, cases are frequently seen in which the plasma protein binding of peptides is low and desirable biostability is not maintained, which leads to the problem of being able to predict the biostability, a different problem to that of conventional small molecule drug discovery.

Technology disclosed in JP-A No. 2017-037378, WO No. 2003/054743, Japanese National-Phase Publication Nos. 2020-523010, or 2020-519246, as listed above, is technology to execute a molecular dynamics simulation of a biopolymer, technology to predict a three dimensional structure of a protein using a computer, and technology to predict a peptide that is effective as a neoantigen, and is not technology for predicting biostability of a peptide. The technology in the citations above accordingly has the problem of not being able to predict peptide biostability.

In consideration of the above circumstances, an object of the present disclosure is to predict biostability of a peptide.

SUMMARY

A prediction device, a prediction method, and a recording medium recorded with a prediction program of a first aspect of the present disclosure are configured to extract a predictive feature vector expressing a feature from a peptide that is a target for biostability prediction, to adjust such that a length of the extracted predictive feature vector is a prescribed length, and to generate a predicted value of biostability for the prediction target peptide by inputting the length-adjusted predictive feature vector into a trained model pre-trained to output a predicted value of peptide biostability from a feature vector expressing a feature of a peptide.

A trained model generation device, a trained model generation method, and a recording medium recorded with a trained model generation program according to a second aspect of the present disclosure are configured to extract a training feature vector expressing a feature from each of plural training peptides, to adjust such that a length of each training feature vector for each of the plural extracted training peptides is a prescribed length, to generate a trained model for outputting a predicted value for peptide biostability from a feature vector expressing a feature of a peptide by executing a machine learning algorithm based on training data that is the length-adjusted training feature vectors paired with correct values of biostability for the training peptides.

A prediction device, a prediction method, and a recording medium recorded with a prediction program of a third aspect of the present disclosure are configured to extract each predictive feature vector expressing a feature from a cyclic peptide that is a target for biostability prediction for instances in which each of plural residues contained in the cyclic peptide is at a start point of a cyclic sequence, and to generate a predicted value for biostability of the prediction target cyclic peptide by inputting the extracted plural predictive feature vectors into a trained model pre-trained to output a predicted value of peptide biostability from a feature vector expressing a feature of a cyclic peptide.

A trained model generation device, a trained model generation method, and a recording medium recorded with a trained model generation program of a fourth aspect of the present disclosure are configured to extract a training feature vector expressing a feature from out of plural training cyclic peptides for instances in which each of plural residues contained in the respective training cyclic peptide is at a start point of a cyclic sequence, to generate a trained model for outputting a predicted value of biostability of a cyclic peptide from a feature vector expressing a feature of a cyclic peptide by executing a machine learning algorithm based on training data that is the plural extracted training feature vectors for each of plural training cyclic peptides paired with a correct value of biostability for the respective training cyclic peptide.

A trained model generation device, a trained model generation method, and a recording medium recorded with a trained model generation program of a fifth aspect of the present disclosure are configured to extract a first training feature vector expressing a feature from each of plural training cyclic peptides, to generate plural second training feature vectors for each of the extracted first training feature vectors by cyclically shifting elements of the first training feature vector, to generate training data expressed by the first training feature vector and the plural second training feature vectors paired with a correct value for biostability of the respective training cyclic peptide, and to generate a trained model for outputting a predicted value of biostability of a cyclic peptide from a feature vector expressing a feature of a cyclic peptide by executing a machine learning algorithm based on the plural generated training data.

A prediction device, a prediction method, and a recording medium recorded with a prediction program according to a sixth aspect of the present disclosure are configured to extract a predictive feature vector expressing a feature from a cyclic peptide that is a target for biostability prediction, and to generate a predicted value of biostability for the prediction target peptide by inputting the extracted predictive feature vector into the trained model generated by the trained model generation device, the trained model generation method, or the trained model generation program of the fifth aspect.

A trained model prediction device, a trained model prediction method, and a recording medium recorded with a trained model prediction program according to a seventh aspect of the present disclosure are configured to generate a trained convolutional neural network model for outputting a predicted value of biostability of a cyclic peptide from a feature vector expressing a feature of a cyclic peptide by, based on training data expressed by a training feature vector expressing a feature extracted from each of plural training cyclic peptides paired with a correct value of biostability for the plural respective training cyclic peptides, executing a machine learning algorithm employing a convolutional neural network model including a both-end-adjacency layer in which elements at both ends of the training feature vector are placed adjacent to one another.

A prediction device, a prediction method, and a recording medium recorded with a prediction program according to an eighth aspect of the present disclosure are configured to extract a predictive feature vector expressing a feature from a cyclic peptide that is a target for biostability prediction, and to generate a predicted value of biostability of the prediction target peptide by inputting the extracted predictive feature vector into a trained convolutional neural network model including a both-end-adjacency layer in which elements at both ends of a feature vector expressing a feature of a cyclic peptide are placed adjacent to one another, the trained convolutional neural network model being configured to output a predicted value of biostability of a peptide from the feature vector.

A prediction device, a prediction method, and a recording medium recorded with a prediction program according to a ninth aspect of the present disclosure are configured to configured to generate plural conformations adoptable by a peptide that is a target for biostability prediction, to select a conformation to be subjected to docking calculation from out of the plural generated conformations based on a prescribed selection criteria, and to predict a predicted value of biostability of the prediction target peptide by performing docking calculation between the prediction target peptide corresponding to the selected conformation and a blood plasma protein.

A prediction device, a prediction method, and a recording medium recorded with a prediction program according to a tenth aspect of the present disclosure are configured to compute a predicted value of a first biostability expressing an biostability of a peptide that is a target for biostability prediction by performing docking calculation between the peptide and a blood plasma protein, to generate a predicted value of a second biostability expressing an biostability of the peptide by inputting a feature vector extracted from the prediction target peptide into a trained model generated in advance by a machine learning algorithm, and to compute a predicted value of biostability of the peptide by consolidating the first generated biostability predicted value with the second generated biostability predicted value.

A prediction device, a prediction method, and a recording medium recorded with a prediction program according to an eleventh aspect of the present disclosure are configured to compute a docking profile including a docking score between a peptide that is the target for biostability prediction and a blood plasma protein by performing docking calculation between the peptide and the blood plasma protein, and to generate a predicted value of biostability of the prediction target peptide by inputting a predictive feature vector including the computed docking profile into a trained model generated in advance by a machine learning algorithm.

A trained model prediction device, a trained model prediction method, and a recording medium recorded with a trained model prediction program according to a twelfth aspect of the present disclosure are configured to compute a training docking profile that is a docking profile including a docking score of a training peptide by performing docking calculation between plural training peptides and a blood plasma protein, and to generate a trained model for outputting a predicted value of biostability of a peptide from a feature vector including a docking profile obtained by performing docking calculation of the peptide by executing a machine learning algorithm based on training data expressed by a training feature vector including a computed training docking profile for each of plural training peptides paired with a correct value of biostability for the respective training peptides.

A prediction device, a prediction method, and a recording medium recorded with a prediction program according to a thirteenth aspect of the present disclosure are configured to extract a feature value from a peptide that is a target for biostability prediction, to identify types of residue in the prediction target peptide, to read, from a storage section stored with docking calculation results of the residues for each of plural types of residue, a docking calculation result corresponding to the types of residue identified, and to predict the biostability of the peptide by inputting a predictive feature vector including the read docking calculation results for the prediction target residue and an extracted feature value into a trained model generated in advance using a machine learning algorithm.

The present disclosure obtains the advantageous effect of being able to predict biostability of a peptide.

The object and advantages of the invention will be realized and attained by means of the elements and combinations particularly pointed out in the claims.

It is to be understood that both the foregoing general description and the following detailed description are exemplary and explanatory and are not restrictive of the invention.

## BRIEF DESCRIPTION OF THE DRAWINGS

Fig. 1 is a block diagram illustrating a prediction device according to a first exemplary embodiment.
Fig. 2 is a diagram illustrating an example of data stored in a data storage section 12.
Fig. 3A is a diagram to explain a cyclic peptide.
Fig. 3B is a diagram to explain a structure of a cyclic peptide.
Fig. 4A is a diagram illustrating an example of training data stored in a training data storage section 16.
Fig. 4B is a diagram to explain a trained model.
Fig. 5 is a diagram illustrating a computer to implement a prediction device according to the first exemplary embodiment.
Fig. 6 is a diagram illustrating an example of a trained model generation processing routine executed in a prediction device according to the first exemplary embodiment.
Fig. 7 is a diagram illustrating an example of a prediction processing routine executed in a prediction device according to the first exemplary embodiment.
Fig. 8 is a block diagram illustrating a prediction device according to a second exemplary embodiment.
Fig. 9 is a diagram illustrating an example of a trained model generation processing routine executed in a prediction device according to the second exemplary embodiment.
Fig. 10 is a diagram illustrating an example of a prediction processing routine executed in a prediction device according to the second exemplary embodiment.
Fig. 11 is a block diagram illustrating a prediction device according to a third exemplary embodiment.
Fig. 12 is a diagram to explain generation of second training feature vectors.
Fig. 13 is a configuration diagram of a conventional convolutional neural network model.
Fig. 14 is a configuration diagram of a convolutional neural network model of a fourth exemplary embodiment.
Fig. 15 is a diagram illustrating a manner of binding between a peptide and a blood plasma protein.
Fig. 16 is a diagram illustrating a manner of binding between a peptide and a blood plasma protein.
Fig. 17 is a block diagram illustrating a prediction device according to a fifth exemplary embodiment.
Fig. 18 is a diagram illustrating an example of a prediction processing routine executed in a prediction device according to the fifth exemplary embodiment.

Fig. 19 is a block diagram illustrating a prediction device according to a sixth exemplary embodiment.

Fig. 20 is a block diagram illustrating a prediction device according to a seventh exemplary embodiment.

Fig. 21 is a diagram illustrating an example of a trained model generation processing routine executed in a prediction device according to the seventh exemplary embodiment.

Fig. 22 is a diagram illustrating an example of a prediction processing routine executed in a prediction device according to the seventh exemplary embodiment.

Fig. 23 is a block diagram illustrating a prediction device according to an eighth exemplary embodiment.

Fig. 24 is a diagram illustrating an example of a prediction processing routine executed in a prediction device according to the eighth exemplary embodiment.

DETAILED DESCRIPTION

Detailed explanation follows regarding exemplary embodiments of the present invention, with reference to the drawings.

First Exemplary Embodiment

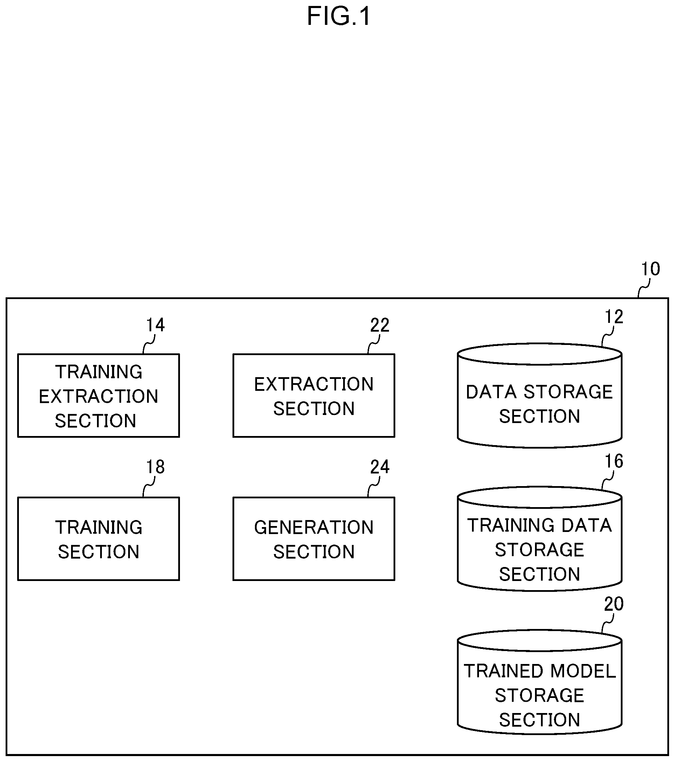

Fig. 1 is a block diagram illustrating an example of a configuration of a prediction device 10 according to a first exemplary embodiment. As illustrated in Fig. 1, in terms of functionality, the prediction device 10 includes a data storage section 12, a training extraction section 14, a training data storage section 16, a training section 18, a trained model storage section 20, an extraction section 22, and a generation section 24.

The prediction device 10 of the present exemplary embodiment predicts biostability of a cyclic peptide.

Training peptide information expressing cyclic peptides used for training and correct values for biostability of these training cyclic peptides are stored associated with each other in the data storage section 12. Note that the peptide information is information including at least one type of information from out of a chemical formula of the peptide, SMILES notation of the peptide, a primary structure of the peptide, a secondary structure of the peptide, a tertiary structure of the peptide, or a quaternary structure the peptide.

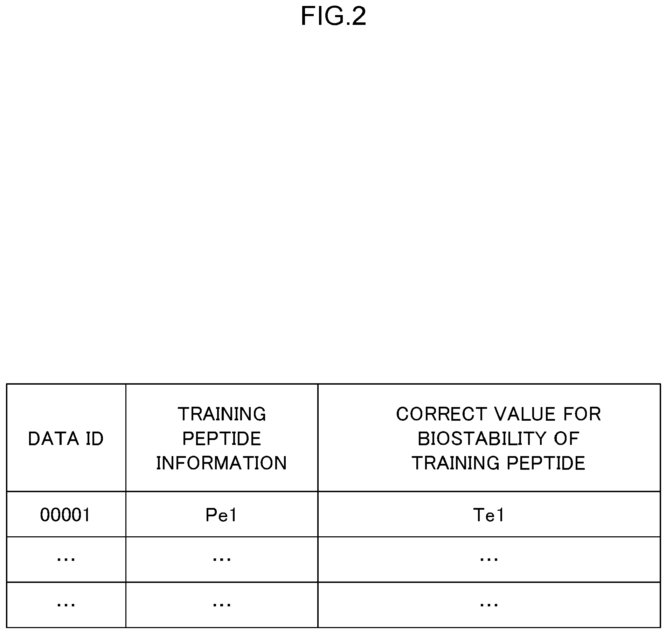

The correct values for biostability of the training cyclic peptides are, for example, data obtained by performing known experiment on the training cyclic peptides. Fig. 2 illustrates an example of data stored in the data storage section 12. As illustrated in Fig. 2, the training peptide information and the correct values for biostability of the training cyclic peptides are stored associated with each other in the data storage section 12.

The training extraction section 14 extracts training feature vectors expressing features of cyclic peptides from the plural training peptide information stored in the data storage section 12. Note that the feature vectors are extracted from the peptide information using a known method.

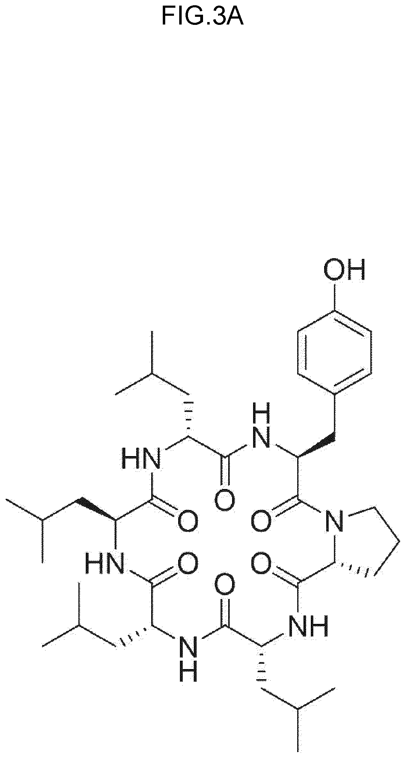

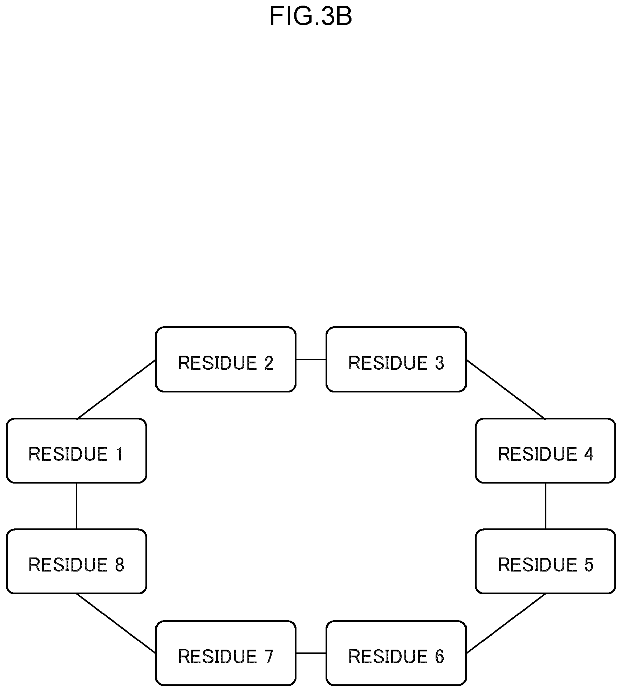

Fig. 3A and Fig. 3B are diagrams for explaining the structure of a cyclic peptide. Fig. 3A is a diagram illustrating an example of a cyclic peptide. The cyclic peptide illustrated in Fig. 3A includes plural residues, and a ring is formed by these residues. Fig. 3B schematically illustrates a configuration of a cyclic peptide. When configuring the feature vectors of the cyclic peptide by extracting an overall feature vector of the cyclic peptide and a feature vector of each residue from a cyclic peptide such as that illustrated in Fig. 3B, the feature vector configuration differs depending on which residue is at a start point of the cyclic sequence.

For example, for a feature vector configuration in which the residue 1 illustrated in Fig. 3B is at the start point of the cyclic sequence, the feature vector [F1, F2,... F8] is configured with the feature value F1 extracted from the residue 1 at the start point. On the other hand, for a feature vector configuration in which the residue 8 is at the start point of the cyclic sequence, the feature vector [F8, F1, F2... F7] is extracted with the feature value F8 extracted from the residue 8 at the start point.

Thus, even if the cyclic peptides are the same, the feature vectors will be different in cases in which the residue at the start point of the cyclic sequence is different. The biostability of cyclic peptides is not able to be appropriately predicted for such cases.

To address this, in the present exemplary embodiment, the respective feature vectors are extracted for instances in which each of the plural residues contained in the cyclic peptide is at the start point of the cyclic sequence, and the biostability is predicted based on these plural feature vectors.

Specifically, from peptide information for each of plural items of training cyclic peptides, the training extraction section 14 extracts feature vectors expressing features for instances in which each of the plural residues contained in the training cyclic peptide is at the start point of the cyclic sequence.

For example, the training extraction section 14 extracts a feature vector 1 for an instance in which the residue 1 illustrated in Fig. 3B is at the start point of the cyclic sequence, extracts a feature vector 2 for an instance in which the residue 2 is at the start point of the cyclic sequence, and so on until it extracts a feature vector 8 for an instance in which

the residue 8 is at the start point of the cyclic sequence.

The training extraction section 14 sets each single extracted feature vector as a single training feature vector. Thus, a set of feature vectors extracted from a single training cyclic peptide corresponds to a training feature vector set.

For each of the plural training cyclic peptides, the training extraction section 14 associates the training feature vector set with a correct value for biostability of the training peptide, and stores these in the training data storage section 16.

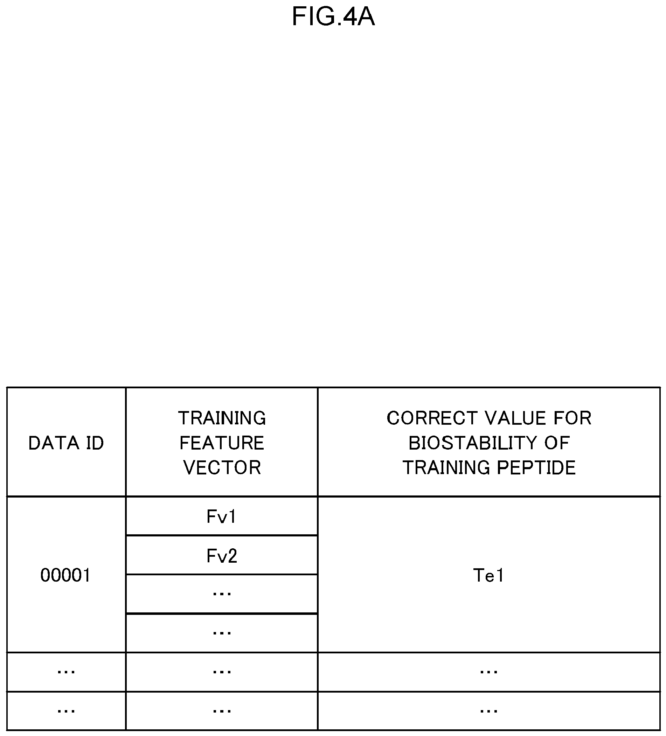

Plural training data are stored in the training data storage section 16. A single item of training data is training feature vectors paired with a correct value for biostability of the training peptide. Fig. 4A illustrates an example of the training data stored in the training data storage section 16. As illustrated in Fig. 4A, the training feature vectors and the correct values for biostability of the training peptides are stored associated with each other in the training data storage section 16. This training data is employed to generate a trained model, described later. Note that the plural training feature vectors Fv1, Fv2, etc. in the example in Fig. 4A are training feature vectors obtained by employing different start points for the cyclic sequence.

The training section 18 generates a trained model, for outputting a predicted value for peptide biostability from feature vectors, by executing a known supervised machine learning algorithm based on the plural training data stored in the training data storage section 16. The training section 18 then stores the trained model in the trained model storage section 20. Note that the trained model itself is a known model, and may for example be a neural network model, a support vector machine, a logistic regression model, or the like. Note that neural network models include deep neural network models obtained by deep learning.

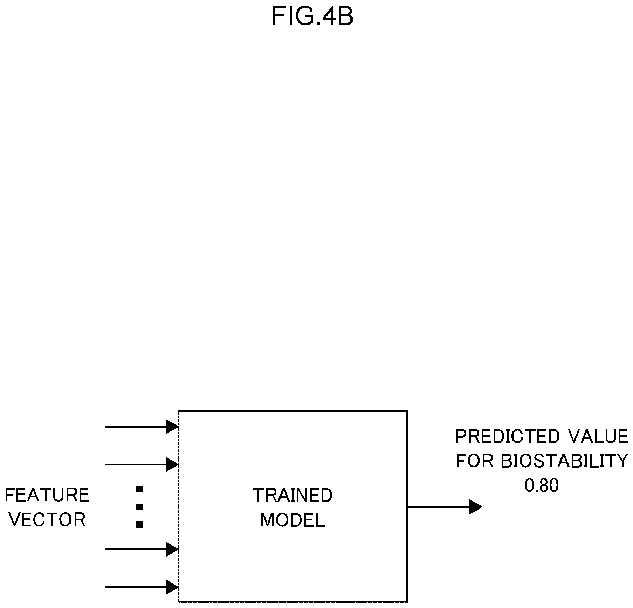

Fig. 4B is a diagram for explaining a trained model. As illustrated in Fig. 4B, when feature vectors extracted from a cyclic peptide that is a target for biostability prediction are input into the trained model, a predicted value is output for the biostability of the prediction target cyclic peptide.

Note that, as described below, plural feature vectors are also extracted from the cyclic peptide that is the biostability prediction target by employing different start points for the cyclic sequence. By inputting each of these plural feature vectors into the trained model a predicted value of biostability is obtained corresponding to each of the plural feature vectors.

The trained model generated by the training section 18 is stored in the trained model storage section 20. Note that the trained model is data in which a structure and trained parameters of a model are associated with each other.

The extraction section 22 extracts feature vectors expressing features from the biostability prediction target cyclic peptide. Specifically, from the peptide information regarding the biostability prediction target cyclic peptide, the extraction section 22 extracts respective feature vectors (hereafter referred to as predictive feature vectors) expressing features for instances in which each of the plural residues contained in the cyclic peptide is at the start point of the cyclic sequence.

The generation section 24 generates a predicted value of biostability for the prediction target cyclic peptide by inputting the plural predictive feature vectors obtained by the extraction section 22 into the trained model stored in the trained model storage section 20.

Specifically, the generation section 24 generates respective predicted values for biostability of the prediction target peptide by inputting each of plural predictive feature vectors obtained by the extraction section 22 into the trained model. Note that a single predicted value corresponds to a single predictive feature vector. The generation section 24 then generates a representative value for the plural predicted values and sets the representative value as the biostability of the prediction target peptide. For example, the generation section 24 may generate an average value of the plural predicted values as the representative value. Alternatively, the generation section 24 may generate a maximum value or a minimum value of the plural predicted values as the representative value.

Note that either the plural predicted values or the representative value for biostability generated by the generation section 24 may be displayed on a display section (not illustrated in the drawings).

In this manner, the prediction device 10 of the first exemplary embodiment extracts respective feature vectors for instances in which each of the plural residues contained in a cyclic peptide is at the start point of the cyclic sequence, and predicts biostability based on these plural feature vectors. This obtains plural feature vectors in consideration of rotational symmetry of the cyclic peptide, and enables biostability of the cyclic peptide to be predicted in an appropriate manner based on these feature vectors.

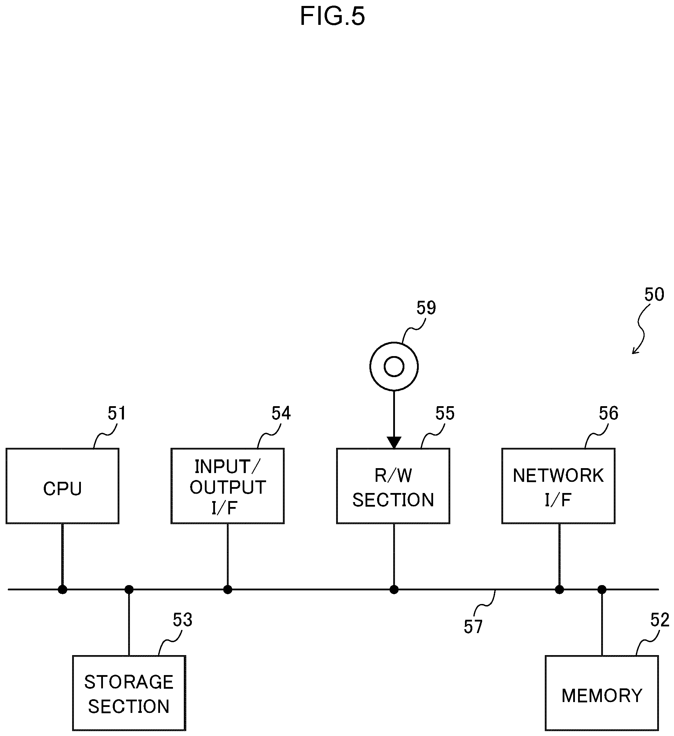

The prediction device 10 may for example be implemented by a computer 50 such as that illustrated in Fig. 5. The computer 50 implementing the prediction device 10 includes a CPU 51, memory 52 serving as a temporary storage area, and a non-volatile storage section 53. The computer 50 also includes an input/output interface (I/F) 54 to which an input/output device or the like (not illustrated in the drawings) is connected, and a read/write (R/W) section 55 that controls reading of data from, and writing of data to, a recording medium 59. The computer 50 also includes a network I/F 56 connected to a network such as the internet. The CPU 51, the memory 52, the storage section 53, the input/output I/F 54, the R/W section 55, and the network I/F 56 are connected to each other through a bus 57.

The storage section 53 may be implemented by a hard disk drive (HDD), a solid state drive (SSD), flash memory, or the like. The storage section 53 serves as a storage medium and is stored with a program causing the computer 50 to function. The CPU 51 reads the program from the storage section 53, expands the program in the memory 52, and sequentially execute processes in the program.

Next, explanation follows regarding operation of the prediction device 10 of the first exemplary embodiment.

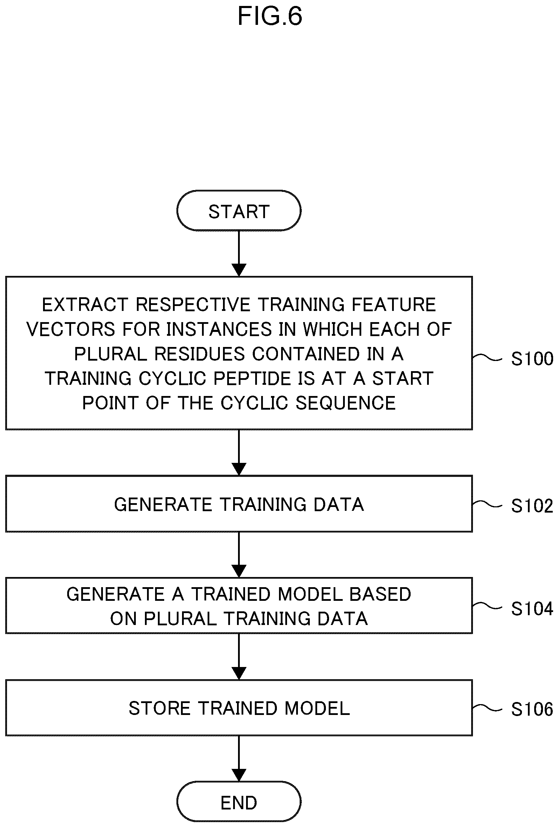

On receiving an instruction signal indicating an instruction to perform trained model generation processing, the prediction device 10 executes a trained model generation processing routine as illustrated in Fig. 6.

At step S100, the training extraction section 14 extracts, from each of the peptide information for plural training cyclic peptides, the training feature vectors expressing features for the instances in which each of the plural residues contained in a training cyclic peptide is at the start point of the cyclic sequence.

At step S102, the training extraction section 14 associates the set of training feature vectors extracted at step S100 with a correct value for biostability of the training cyclic peptide to generate training data, and temporarily stores this training data in the training data storage section 16.

At step S104, the training section 18 generates a trained model, for outputting a predicted value for peptide biostability from feature vectors, by executing a known supervised machine learning algorithm based on the plural training data stored in the training data storage section 16.

At step S106, the training section 18 stores the trained model generated at step S104 in the trained model storage section 20.

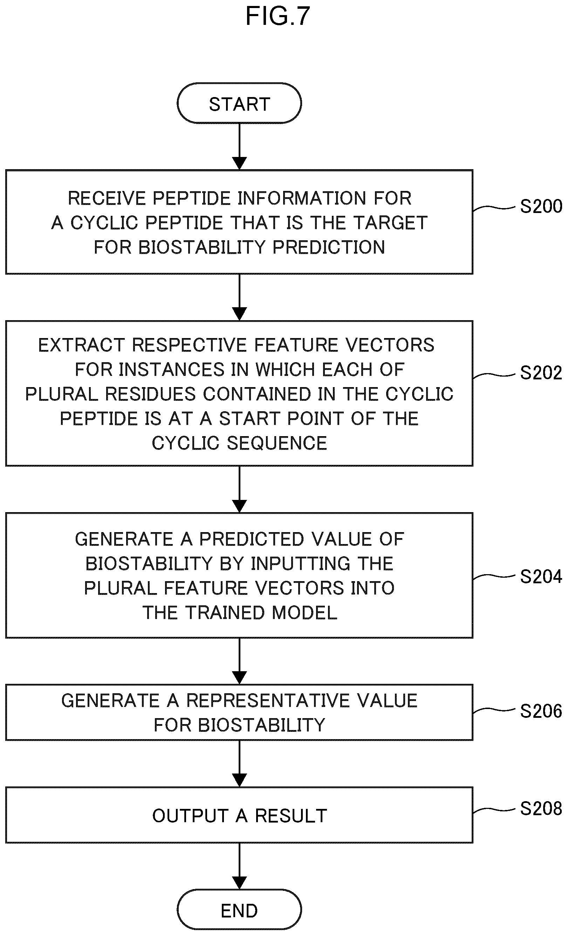

When the trained model has been stored in the trained model storage section 20 and the peptide information for the biostability prediction target has been input to the prediction device 10, the prediction device 10 executes the prediction processing routine illustrated in Fig. 7.

At step S200, the extraction section 22 receives the peptide information for the biostability prediction target.

At step S202, from the peptide information received at step S200, the extraction section 22 extracts respective predictive feature vectors expressing features for instances in which each of the plural residues contained in the cyclic peptide is at the start point of the cyclic sequence.

At step S204, the generation section 24 generates plural predicted values of biostability for the prediction target peptide by inputting each of the plural predictive feature vectors extracted at step S202 into the trained model stored in the trained model storage section 20.

At step S206, the generation section 24 generates a representative value from the plural predicted values generated at step S204.

At step S208, the generation section 24 outputs the representative value of the predicted values of biostability generated at step S206 as a result.

As described in detail above, the prediction device of the first exemplary embodiment extracts from each of plural training cyclic peptides a set of training feature vectors expressing features for instances in which each of plural residues contained in the training cyclic peptide is at the start point of the cyclic sequence. Then, for each of the plural training cyclic peptides, the prediction device executes a machine learning algorithm based on the training data that is the extracted plural training feature vectors paired with correct values for the biostability of the training cyclic peptides, so as to generate a trained model for outputting a predicted value of biostability for a cyclic peptide from feature vectors expressing cyclic peptide features. This enables a trained model to be obtained for predicting the biostability of cyclic peptides. Note that the trained model is trained based on training feature vectors for instances in which each of the plural residues is at the start point of the cyclic sequence, and so the model is suited to predicting the biostability of cyclic peptides.

Moreover, the prediction device of the first exemplary embodiment extracts from a biostability prediction target cyclic peptide respective feature vectors expressing features for instances in which each of plural residues contained in the cyclic peptide is at the start point of the cyclic sequence. The prediction device then generates a predicted value of biostability for the prediction target cyclic peptide by inputting the plural feature vectors into the trained model. This enables the biostability of the cyclic peptide to be predicted. Specifically, as described previously, the trained model is trained based on the training feature vectors for instances in which each of the plural residues is at the start point of the cyclic sequence, and so the model is suited to predicting the biostability of cyclic peptides. This enables predicted values for biostability to be generated in consideration of the cyclic peptide structure.

Second Exemplary Embodiment

Next, explanation follows regarding a second exemplary embodiment. A prediction device of the second exemplary embodiment differs from the first exemplary embodiment in that lengths of the plural feature vectors are aligned. Note that although an example of a case applied to cyclic peptides as the target has been described in the first exemplary embodiment, there is no limitation to cyclic peptides in the second exemplary embodiment, and linear peptides may be the target. Moreover, similar portions in the configuration of the prediction device according to the second exemplary embodiment to those of the prediction device of the first exemplary embodiment are allocated the same reference numerals, and explanation thereof is omitted.

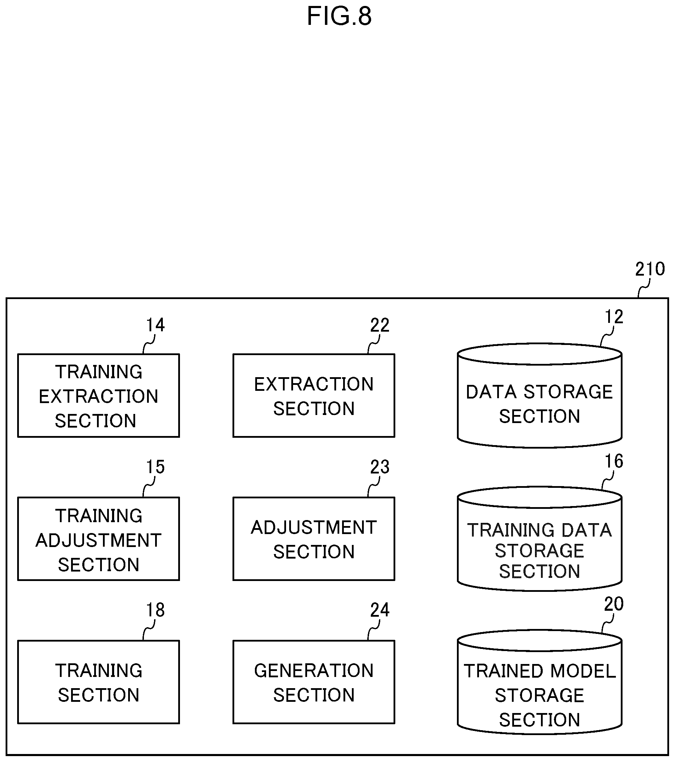

Fig. 8 is a block diagram illustrating an example of a configuration of a prediction device 210 according to the second exemplary embodiment. As illustrated in Fig. 8, in terms of functionality, the prediction device 210 includes the data storage section 12, the training extraction section 14, the training data storage section 16, a training adjustment section 15, the training section 18, the trained model storage section 20, the extraction section 22, an adjustment section 23, and the generation section 24.

The training adjustment section 15 performs adjustment such that the respective lengths of the training feature vectors of the plural training peptides extracted by the training extraction section 14 become a prescribed length.

The peptides includes plural residues. Thus the length of the feature vectors differs between peptides that have a different number of residues. Specifically, the number of feature vector elements correspond to the number of residues, and so the length of the feature vectors differs between peptides that have a different number of residues. Note that the length of feature vectors input into a trained model such as a neural network model are preferably uniform. For example, in cases in which the number of feature vector elements is ten, an action is required to make it such that there is also a corresponding ten nodes in the input layer of the neural network model, an example of a trained model.

Thus, in cases in which the lengths of the feature vectors extracted from each of the plural peptides differ, unless some appropriate measure is taken, a trained model employing a machine learning algorithm such as neural network model cannot be built, or the peptide biostability cannot be predicted using such a trained model.

To address this, in the prediction device of the second exemplary embodiment, the lengths of the feature vectors extracted from the peptides are aligned, thereby enabling training to be performed using a machine learning algorithm that employs these feature vectors. Furthermore, the peptide biostability can be predicted using a trained model obtained by training.

Specifically, for example, the training adjustment section 15 identifies the training feature vector with the maximum length from out of the plural training feature vectors, and performs adjustment such that the lengths of the plural other training feature vectors become this maximum length. Alternatively, for example, the training adjustment section 15 may perform adjustment such that the respective lengths of the plural training feature vectors become a prescribed length. Note that the prescribed length in such cases may be preset by a user.

For example, the training adjustment section 15 may align the length of the training feature vectors by converting using a known padding method. A padding method is a method in which a vacant location of a target is filled with a substitute value or the like. Thus, for example, in the case of a training feature vector [0.13, 0.45, 0.82] with a length of three, if the prescribed length is five then the training adjustment section 15 may use a padding method so as to generate a training feature vector with a length of five such as [0.00, 0.13, 0.45, 0.82, 0.00]. Note that when adjusting the lengths of the training feature vectors, the training adjustment section 15 may add an element containing information about the length pre-adjustment, such as the residue number prior to length adjustment.

Alternatively, for example, the training adjustment section 15 may align the lengths of the training feature vectors by conversion using a linear interpolation method. Specifically, the training adjustment section 15 may compute a feature value x', this being an element of a training feature vector, using the following Equation (1).

$$j' = \left\lceil \frac{(k-1)(j-1)}{(m-1)} \right\rceil + 1$$

$$x'_j = \frac{(n-1)(j-1)-(m-1)(j'-1)}{(m-1)(k-1)}(x_{j'+1} - x_{j'}) + x_{j'}$$

$$(1)$$

Wherein: $x_i$ is a feature value of a residue position i of a peptide with residue length k ($1 \leq i \leq k$); and $x'_j$ is a $j^{th}$ feature value of sequence length m after interpolation ($1 \leq j \leq m$).

The training adjustment section 15 converts a training feature vector with a length k obtained from a peptide with a residue length k into a training feature vector with a length m according to the Equation (1). Note that $x_i$ is a feature value at the position of an $i^{th}$ element of a training feature vector x prior to conversion, and $x'_j$ is a feature value at the position of an $j^{th}$ element of a training feature vector x' after conversion. The lengths of plural training feature vectors are aligned in this manner.

The training adjustment section 15 then associates the training feature vectors having aligned lengths with the correct values for biostability of the corresponding training peptides, and stores these in the training data storage section 16.

There are plural training data stored in the training data storage section 16.

The training section 18 generates a trained model, for outputting a predicted value for peptide biostability from feature vectors, by executing a known supervised machine learning algorithm based on the plural training data stored in the training data storage section 16. The training section 18 then stores the trained model in the trained model storage section 20.

The trained model generated by the training section 18 is stored in the trained model storage section 20.

The extraction section 22 extracts predictive feature vectors expressing features from the biostability prediction target cyclic peptide.

The adjustment section 23 performs adjustment such that the lengths of the predictive feature vectors extracted by the extraction section 22 are the same prescribed length as those in the training data. Specifically, the adjustment section 23 adjusts the lengths of the predictive feature vectors using a similar method to the training adjustment section 15 as described above.

The generation section 24 generates a predicted value for biostability of the prediction target peptide by inputting the predictive feature vectors with their lengths adjusted by the adjustment section 23 into the trained model stored in the trained model storage section 20.

Note that the predicted values for biostability generated by the generation section 24 are displayed on a display section (not illustrated in the drawings).

Next, explanation follows regarding operation of the prediction device 210 of the second exemplary embodiment.

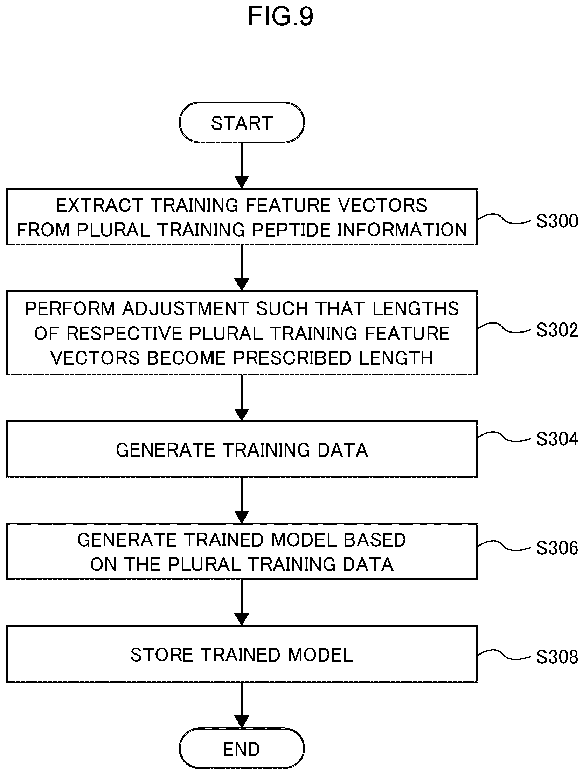

On receiving an instruction signal indicating an instruction to perform trained model generation processing, the prediction device 210 executes the trained model generation processing routine illustrated in Fig. 9.

At step S300, the training extraction section 14 extracts training feature vectors expressing features of the training peptide from the plural training peptide information stored in the data storage section 12.

At step S302, the training adjustment section 15 performs adjustment such that the respective lengths of the training feature vectors for the plural training peptides extracted at step S300 become a prescribed length.

At step S304, the training adjustment section 15 associates the training feature vectors having lengths aligned at step S302 with respective correct values for biostability of the training peptides and generates training data to be temporarily stored in the training data storage section 16.

At step S306, the training section 18 generates a trained model, for outputting a predicted value for peptide biostability from feature vectors expressing peptide features, by executing a known supervised machine learning algorithm based on the plural training data stored in the training data storage section 16.

At step S308, the training section 18 stores the trained model generated at step S306 in the trained model storage section 20.

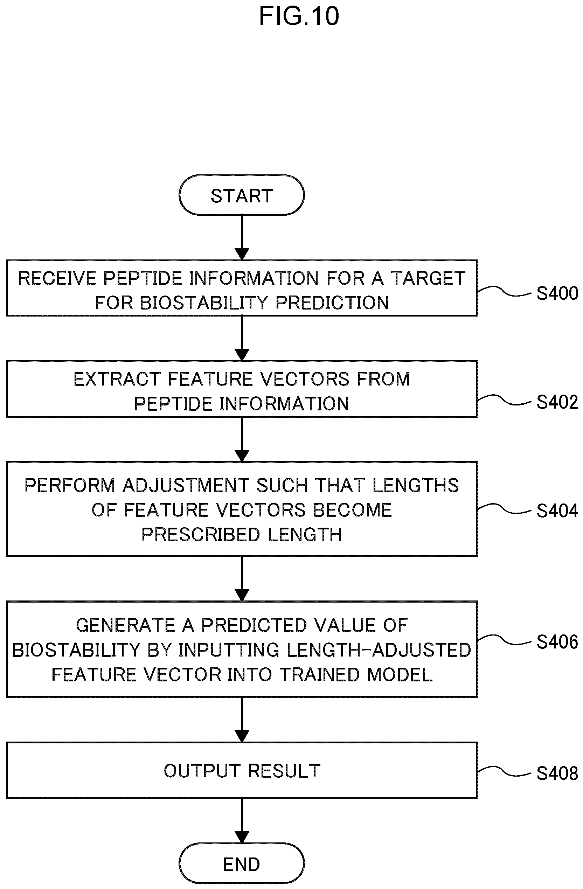

When the trained model has been stored in the trained model storage section 20, and the peptide information for the biostability prediction target has been input to the prediction device 210, the prediction device 210 executes the prediction processing routine illustrated in Fig. 10.

At step S400, the extraction section 22 receives the peptide information for the biostability prediction target.

At step S402, the extraction section 22 extracts predictive feature vectors from the peptide information received at step S400.

At step S404, the adjustment section 23 performs adjustment such that the lengths of the predictive feature vectors extracted at step S402 become the prescribed length.

At step S406, the generation section 24 generates a predicted value of biostability for the prediction target peptide by inputting the predictive feature vectors having lengths adjusted at step S404 into the trained model stored in the trained model storage section 20.

At step S408, the generation section 24 outputs the predicted value for biostability generated at step S406 as a result.

As described in detail above, the prediction device of the second exemplary embodiment performs adjustment such that the respective lengths of the training feature vectors for the plural training peptides become the prescribed length. The prediction device then generates a trained model, for outputting a predicted value for peptide biostability from feature vectors extracted from peptides, by executing a machine learning algorithm based on the training data in which the length-adjusted training feature vectors are paired with the respective correct values for biostability of the training peptides. Thus a trained model can be obtained for predicting peptide biostability, even in cases in which peptides are configured from plural residues having a different number of residues.

Moreover, the prediction device of the second exemplary embodiment generates a predicted value for biostability of a prediction target peptide by adjusting the length of feature vectors extracted from the peptide that is the biostability prediction target so as to become the prescribed length, and inputting the length-adjusted feature vectors into the trained model. This enables the peptide biostability to be predicted even in cases in which peptides are configured from plural residues having a different number of residues.

Third Exemplary Embodiment

Next, explanation follows regarding a third exemplary embodiment. A prediction device of the third exemplary embodiment differs from the first and second exemplary embodiments in respect that the training data is augmented by data augmentation that focuses on the structural properties of a cyclic peptide, and a trained model is generated based on this augmented training data. Note that similar portions in the configuration of the prediction device according to the third exemplary embodiment to those of the prediction devices of the first and second exemplary embodiments are allocated the same reference numerals, and explanation thereof is omitted.

When augmenting the training feature vectors, the prediction device of the third exemplary embodiment performs a similar length adjustment to that in the second exemplary embodiment, and then cyclically shifts elements of the training feature vectors so as to generate plural training feature vectors. This enables the training data to be augmented while considering structural characteristic of the cyclic peptides.

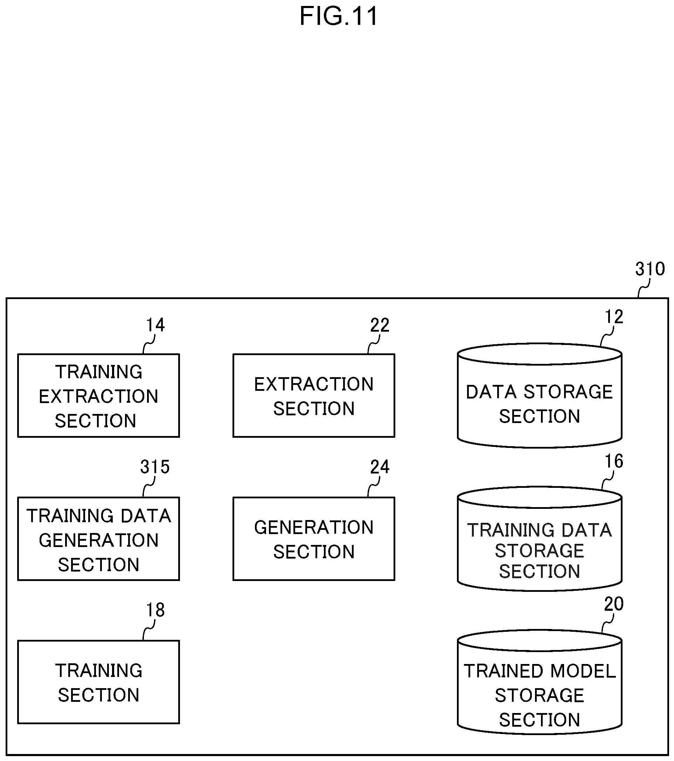

Fig. 11 is a block diagram illustrating an example of a configuration of a prediction device 310 according to the third exemplary embodiment. As illustrated in Fig. 11, in terms of functionality the prediction device 310 includes the data storage section 12, the training extraction section 14, the training data storage section 16, a training data generation section 315, the training section 18, the trained model storage section 20, the extraction section 22, and the generation section 24.

The training extraction section 14 of the third exemplary embodiment extracts a set of first training feature vectors expressing features of a training cyclic peptide from out of each of the plural peptide information regarding training cyclic peptides.

Specifically, first, the training data generation section 315 aligns the lengths of the plural first training feature vectors to a prescribed length, similarly to in the second exemplary embodiment. Next, for each of the first training feature vectors included in the first training feature vector set extracted by the training extraction section 14, the training data generation section 315 cyclically shifts elements of the first training feature vectors to generate a set of second training feature vectors.

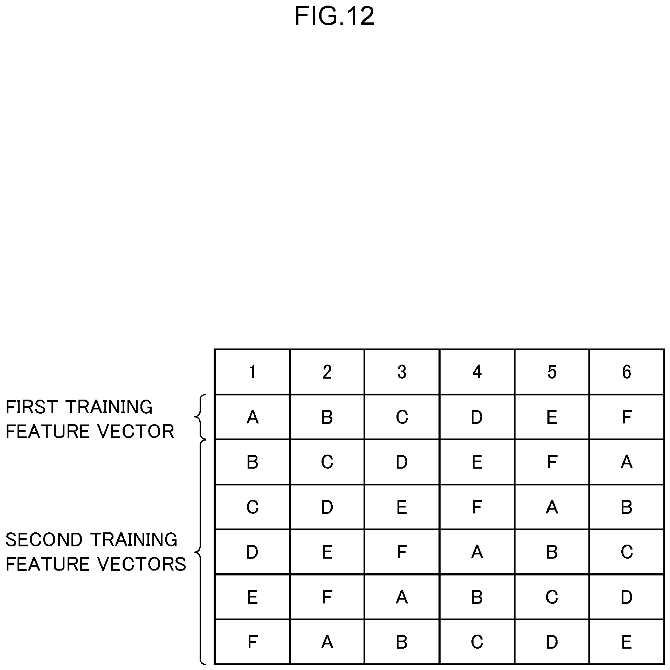

Fig. 12 is a diagram for explaining the generation of second training feature vectors. The number 1 and so on in Fig. 12 indicates positions of elements of the feature vectors. In the example illustrated in Fig. 12, a feature value B may be extracted from a first residue of a given cyclic peptide, a feature value C may extracted from a second residue, a feature value D may extracted from a third residue, and a feature value E may be extracted from a fourth residue. In order to convert a feature vector with a length of four into a feature vector with a length of six, a feature value A is inserted at the location of the number 1, and a feature value F is inserted at the location of the number 6. In this manner, the elements A, B, C, D, E, F become elements of the first training feature vector.

Next, as illustrated in Fig. 12, the training data generation section 315 cyclically shifts the elements A, B, C, D, E, F of the first training feature vector to the left by a distance of one, so as to generate a second training feature vector with the elements B, C, D, E, F, A. Then, in a similar manner, the elements A, B, C, D, E, F of the first training feature vector are cyclically shifted to the left by a distance of two, so as to generate a second training feature vector with the elements C, D, E, F, A, B. During this processing, positions in a sequence are shifted by a fixed distance without changing the order in the sequence between before and after, in a similar manner to rotation processing of a text string or a bit string, in processing that implements a wraparound at an end point. This processing obtains a first training feature vector and plural second training feature vectors from a single cyclic peptide, which can then be employed as training data.

The training data generation section 315 generates training data expressed by the first training feature vector set and the second training feature vectors set paired with respective correct values for biostability of the training cyclic peptides. The training data generation section 315 then stores the plural generated items of training data in the training data storage section 16.

The training section 18 generates a trained model, for outputting a predicted value for biostability of a cyclic peptide from feature vectors expressing cyclic peptide features, by executing a machine learning algorithm based on the plural training data stored in the training data storage section 16.

Note that other configuration and operation of the prediction device 310 of the third exemplary embodiment are similar to those of the first exemplary embodiment or second exemplary embodiment, and so explanation thereof is omitted.

As described above, the prediction device of the third exemplary embodiment extracts the first training feature vectors expressing features from the plural training cyclic peptides. For each of the first training feature vectors, the prediction device adjusts the length of the first training feature vector to a prescribed length, then cyclically shifts the elements of the first training feature vector so as to generate a set of second training feature vectors. The prediction device generates training data expressed by the first training feature vector set and the second training feature vectors set paired with correct values for biostability of the respective training cyclic peptides. The prediction device then generates a trained model, for outputting a predicted value for biostability of a cyclic peptide from feature vectors expressing features of a cyclic peptide, by executing a machine learning algorithm based on the plural generated items of training data. This enables the training data to be augmented while considering structural characteristic of the cyclic peptides. Moreover, the trained model can be obtained based on a large amount of training data generated in consideration of the configuration of the cyclic peptides.

Fourth Exemplary Embodiment

Next, explanation follows regarding a fourth exemplary embodiment. A prediction device of the fourth exemplary embodiment differs from the first to third exemplary embodiments in respect that a predicted value for biostability of a cyclic peptide is generated using a convolutional neural network model including a layer in which elements at both ends of a feature vector are placed adjacent to one another so as to correspond to the structural properties of cyclic peptides. Note that similar portions in the configuration of the prediction device according to the fourth exemplary embodiment to those of any of the prediction devices of the first to third exemplary embodiments are allocated the same reference numerals, and explanation thereof is omitted.

There is a need for feature vectors extracted from a cyclic peptide to be expressed as a ring of the residues configuring the cyclic peptide. In this regard, vectors that are elements simply arrayed in a one-dimensional form result in a start end and a terminal end being created as a result, and thus might not be considered as appropriately expressing the continuity of the ring of residues in a cyclic peptide.

Thus, the prediction device of the fourth exemplary embodiment generates predicted values for biostability of cyclic peptides using a convolutional neural network model including a both-end-adjacency layer in which elements at both ends of a feature vector are placed adjacent to one another. The configuration of the residues of the cyclic peptides are thereby expressed in the convolutional neural network model.

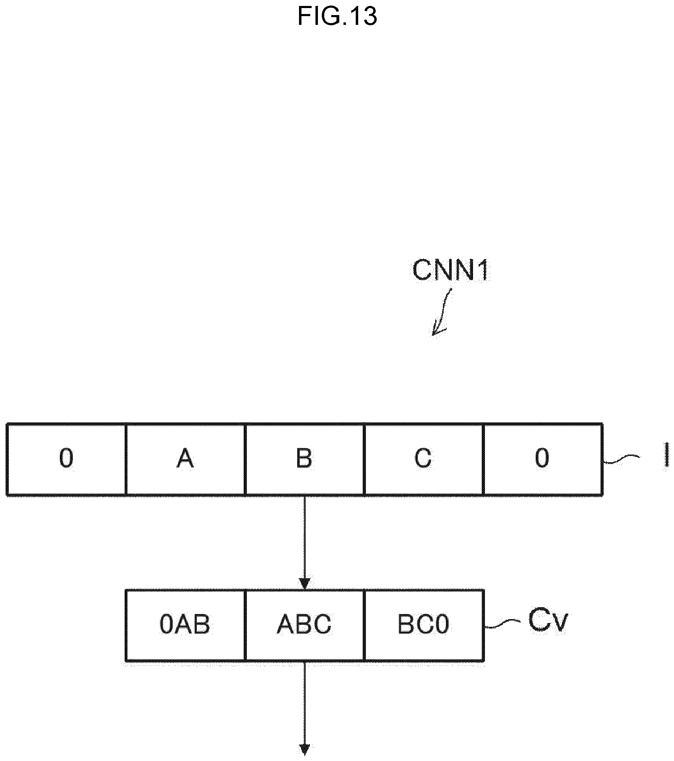

Fig. 13 is a configuration diagram of a conventional convolutional neural network model. As illustrated in Fig. 13, a conventional convolutional neural network model CNN1 includes an input layer I and a convolutional layer Cv. Note that illustration of other convolutional layers, pooling layers, and so on is omitted. As illustrated in Fig. 13, when a feature vector [0, A, B, C, 0] is input to the input layer I, convolutional processing is performed in the convolutional layer Cv such that [0, A, B], [A, B, C], and [B, C, 0] are extracted from the feature vector. However, in this conventional convolutional neural network model CNN1, convolutional processing is merely performed on the input feature vector, and no consideration is made of the structure of the cyclic peptide from which the feature vector was extracted.

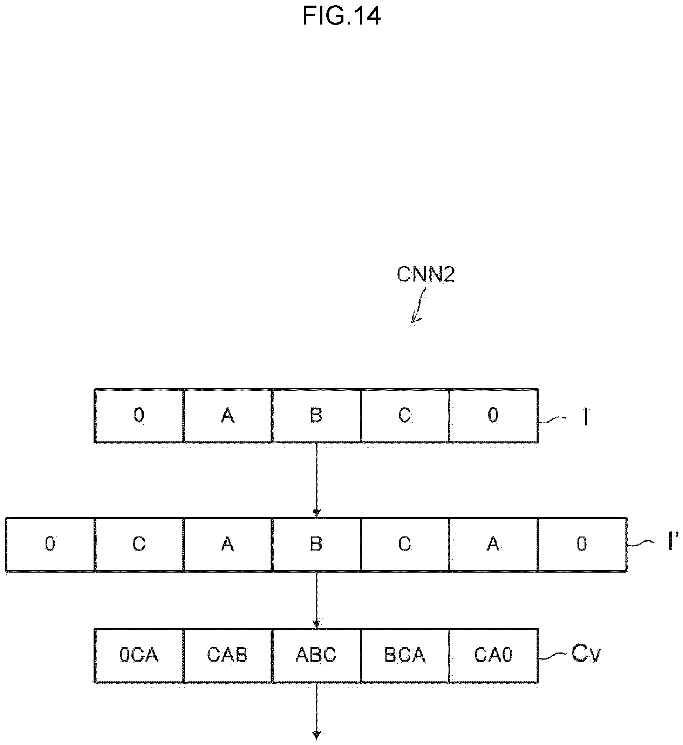

In contrast thereto a convolutional neural network model of the fourth exemplary embodiment includes a layer that considers the structural features of the cyclic peptide. Fig. 14 is a configuration diagram of the convolutional neural network model of the fourth exemplary embodiment. As illustrated in Fig. 14, a convolutional neural network model CNN2 of the fourth exemplary embodiment includes an input layer I, a convolutional layer Cv, and a both-end-adjacency layer I'. The both-end-adjacency layer I' is a layer in which elements at both ends of the feature vector are redisposed so as to be adjacent to each other on the left and right. Specifically, as illustrated in Fig. 14, C is disposed adjacent to the left side of A, and A is disposed adjacent to the right side of C. The ring of residues of the cyclic peptide is expressed in this manner.

Based on plural training data, the training section 18 of the fourth exemplary embodiment, generates a trained convolutional neural network model, for outputting a predicted value for biostability of a cyclic peptide from feature vectors, by training a convolutional neural network model including a both-end-adjacency layer in which elements at both ends of a training feature vector are placed adjacent to one another. The training section 18 then stores the trained convolutional neural network model in the trained model storage section 20.

The generation section 24 of the fourth exemplary embodiment generates predicted values for biostability of prediction target peptides by inputting feature vectors extracted from a biostability prediction target cyclic peptide into the trained convolutional neural network model stored in the trained model storage section 20.

Note that other configuration and operation of the prediction device of the fourth exemplary embodiment are similar to those of a prediction device of the first to third exemplary embodiments, and so explanation thereof is omitted.

As described above, based on plural training data the prediction device of the fourth exemplary embodiment generates a trained convolutional neural network model for outputting a predicted value for biostability of a cyclic peptide from feature vectors, by training a convolutional neural network model including a both-end-adjacency layer in which elements at both ends of training feature vectors are placed adjacent to one another. This enables a trained convolutional neural network model to be obtained that considers structural characteristic of cyclic peptides.

Moreover, the prediction device generates predicted values for biostability of prediction target peptides by inputting feature vectors extracted from a biostability prediction target cyclic peptide into the trained convolutional neural network model including the both-end-adjacency layer in which elements at both ends of a feature vector are placed adjacent to one another. This enables predicted values for biostability to be obtained that consider structural characteristic of cyclic peptides.

Fifth Exemplary Embodiment

Next, explanation follows regarding a fifth exemplary embodiment. A prediction device of the fifth exemplary embodiment differs from the first to fourth exemplary embodiments in respect that predicted values for peptide biostability are generated by executing docking calculation between a peptide and a blood plasma protein. Note that similar portions in the configuration of the prediction device according to the fifth exemplary embodiment to those of any of the prediction devices of the first to fourth exemplary embodiments are allocated the same reference numerals, and explanation thereof is omitted.

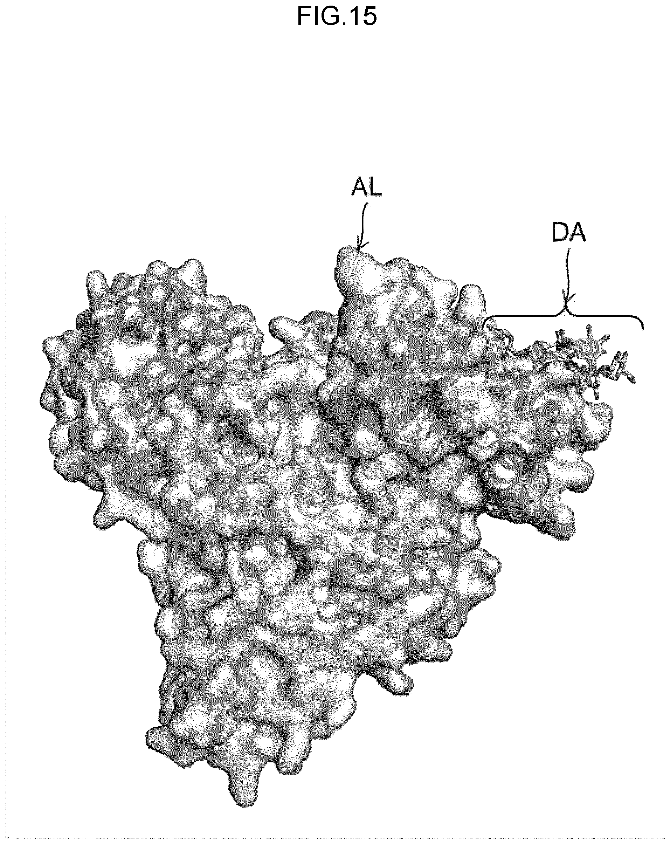

Fig. 15 is a schematic diagram illustrating binding between human serum albumin AL that is an example of a blood plasma protein and dalbavancin DA that is an example of a peptide. Note that the results of research related to Fig. 15 are disclosed in Reference Cited Document 1.

Reference Cited Document 1: Sho Ito, Akinobu Senoo, Satoru Nagatoishi, Masahito Ohue, Masaki Yamamoto, Kouhei Tsumoto, and Naoki Wakui, "Structural Basis for the Binding Mechanism of Human Serum Albumin Complexed with Cyclic Peptide Dalbavancin", J. Med. Chem. 2020, 63, 22, 14045-14053, Publication Date: November 13, 2020.

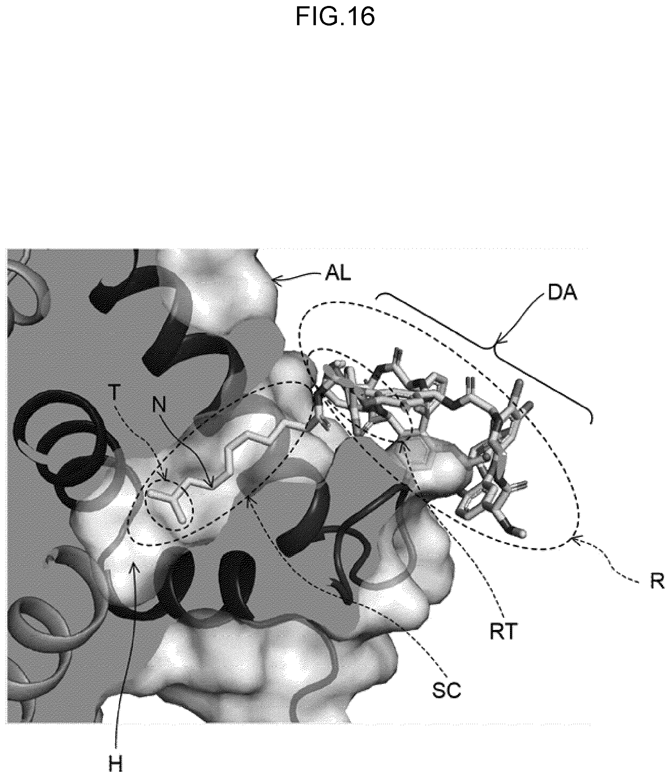

Moreover, Fig. 16 is an expanded diagram of a portion binding between the human serum albumin AL and the dalbavancin DA of Fig. 15. As is apparent from Fig. 16, a side chain SC of the dalbavancin DA is in an state so as to be inserted into a hydrophobic pocket H of the human serum albumin AL. Moreover, it is apparent that a ring portion R of the dalbavancin DA is in a state so as to hang over the human serum albumin AL.

In this manner, a single binding state of the human serum albumin AL with the dalbavancin DA is a binding state as illustrated in Fig. 15 and Fig. 16. From this it is anticipated that in the conformations adoptable by the dalbavancin DA, a state of the side chain SC, a state of a terminal portion T of the side chain SC, a state of a root portion RT of the side chain SC, and the like included in conformations appropriate for binding with the human serum albumin AL are factors affecting biostability.

Thus a prediction device of a fifth exemplary embodiment generates plural conformations adoptable by a peptide that is a target for biostability prediction, and performs a known docking calculation between a peptide and a blood plasma protein for each of the plural conformations.

Note that in the present exemplary embodiment, from out of the plural generated conformations, a conformation is selected that has a high probability of binding with a blood plasma protein, and docking calculation is executed only on the selected conformation. This enables docking calculation to be performed only on conformations having a high probability of binding with a blood plasma protein, instead of performing docking calculation on all of the conformations adoptable by the peptide. This enables docking calculation to be executed efficiently, resulting in being able to efficiently obtain an biostability of the peptide that is the biostability prediction target.

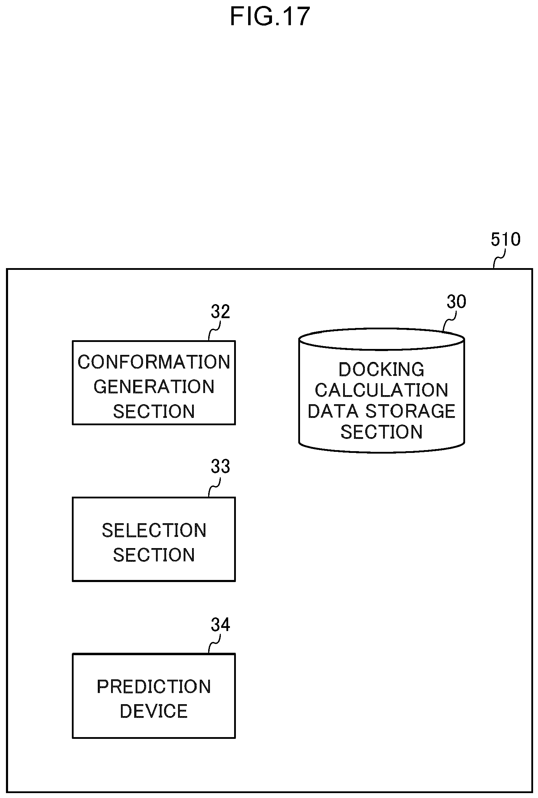

Fig. 17 is a block diagram illustrating an example of a configuration of a prediction device 510 of the fifth exemplary embodiment. As illustrated in Fig. 17, the prediction device 510 is, in terms of functionality, configured including a docking calculation data storage section 30, a conformation generation section 32, a selection section 33, and a prediction device 34.

Various data for executing docking calculation are stored in the docking calculation data storage section 30. The conformation generation section 32, the selection section 33, and the prediction device 34 described later execute docking calculation and predict biostability based on the various data stored in the docking calculation data storage section 30. Note that data obtained from docking calculation are also stored in the docking calculation data storage section 30.

The conformation generation section 32 generates plural conformations adoptable by the peptide that is the biostability prediction target. Specifically, the conformation generation section 32 acquires peptide information for the peptide that is the biostability prediction target stored in the docking calculation data storage section 30. The conformation generation section 32 generates plural ideal conformations adoptable by the peptide based on various information (primary structure of the peptide, secondary structure of the peptide, or tertiary structure of the peptide) included in the peptide information.

Based on a prescribed selection criteria, the selection section 33 selects a conformation to be subjected to docking calculation from out of the plural conformations generated by the conformation generation section 32.

Specifically, the selection section 33 first selects a conformation having a high probability of binding with a blood plasma protein from out of the plural conformations generated by the conformation generation section 32.

As illustrated in Fig. 16, one binding state between the dalbavancin DA and the human serum albumin AL is a case in which the side chain SC of the dalbavancin DA is in a state so as to be inserted into a hydrophobic pocket H of the human serum albumin AL. The length of the side chain of the peptide might accordingly be thought to be a factor affecting biostability. Moreover, a straightness in the side chain of the peptide might also be thought to be an important factor affecting biostability.

In the example illustrated in Fig. 16, a ring portion R of the dalbavancin DA is in a state so as to hang over the human serum albumin AL, and so the structure of the root portion RT of the side chain of the peptide might also be thought to be an important factor affecting biostability.

Moreover, as illustrated in Fig. 16, a three dimensional shape of a terminal portion T of the side chain SC of the dalbavancin DA is also able to correspond to a shape of a deepest portion of the hydrophobic pocket H of the human serum albumin AL, and so the three dimensional shape of the terminal portion of a side chain of a peptide might also be thought to be an important factor affecting biostability. Moreover, the hydrophobic pocket H of the human serum albumin AL is preferably not a charged atom, and so physical conditions, such as whether or not there is a charged atom contained in the side chain, might also be thought to be an important factor.

Thus, for example, from out of the plural conformations generated by the conformation generation section 32, the selection section 33 selects as a conformation having a high probability of binding with a blood plasma protein a conformation of peptide having a length of peptide side chain that is a prescribed value or greater.

Moreover, for example, from out of the plural conformations generated by the conformation generation section 32, the selection section 33 selects as a conformation having a high probability of binding with a blood plasma protein a conformation of peptide having a straightness of peptide side chain that is a prescribed value or greater. Note that, for example, the straightness of the side chain may be computed as having a higher degree of peptide side chain linearity the smaller a total deviation is between an linearity approximation obtained by a least-squares method based on coordinates of plural atoms N of the peptide as illustrated in Fig. 16, and the actual coordinates of the plural atoms N of the peptide.

Moreover, for example, from out of the plural conformations generated by the conformation generation section 32, the selection section 33 selects as a conformation having a high probability of binding with a blood plasma protein a conformation of peptide in which atoms of the root portion RT of the peptide side chain are widely spaced. For example, the selection section 33 selects a conformation of peptide for which the variance of coordinates of the atoms N of the root portion RT of the peptide side chain is a prescribed value or greater.

Moreover, for example, from out of the plural conformations generated by the conformation generation section 32, the selection section 33 selects as a conformation having a high probability of binding with a blood plasma protein a conformation of peptide in which atoms of the tip portion T of the peptide side chain are widely spaced. For example, the selection section 33 a conformation of peptide for which the variance of coordinates of the atoms N of the tip portion T of the peptide side chain is a prescribed value or greater.

Moreover, for example, from out of the plural conformations generated by the conformation generation section 32, the selection section 33 selects as a conformation having a high probability of binding with a blood plasma protein a conformation of peptide in which a physical condition is satisfied, such as the presence or absence of a charged atom in the peptide side chain. This is because, for example, there might be thought to be a low probability of binding with the blood plasma protein in a case in which there is a charged atom in the peptide side chain as illustrated in the example of Fig. 16.

From out of the plural conformations generated by the conformation generation section 32, the selection section 33 selects a conformation satisfying a selection criteria such as those listed above. Note that the selection section 33 may further select a conformation from out of conformations satisfying the selection criteria such as those listed above.

For example, in cases in which plural conformations satisfying the selection criteria such as those listed above are similar conformations to each other, a similar result would be anticipated to be obtained by executing docking calculation when these were selected due to the conformations having low diversity.

Thus, for example, in order for the selection section 33 to select conformations as diverse as possible from out of the plural conformations satisfying the selection criteria such as those listed above, a value is computed for the root mean square deviation (RMSD) of the inter-atomic distances between the conformations satisfying the selection criteria such as those listed above. Note that instead of employing all of the atoms of the peptide, plural atoms of the peptide may, for example, be selected for example, and the RMSD value may be computed based on these atoms alone. The selection section 33 then performs clustering using a known method based on the RMSD values, and further selects one or more conformation from each cluster. This results in the selection of conformations having diversity.

The prediction device 34 predicts an biostability of the prediction target peptide by performing docking calculation between the prediction target peptides corresponding to the conformations selected by the selection section 33 and the blood plasma protein.

Specifically, the prediction device 34 performs docking calculation between each of the prediction target peptides corresponding to each of the plural conformations selected by the selection section 33 and the blood plasma protein. The prediction device 34 then predicts the biostability of the prediction target peptide based on a docking profile that is the docking calculation results obtained for each of the plural conformations selected by the selection section 33. Note that the docking profile is, for example, a vector having elements of a docking score obtained for each residue on the blood plasma protein side. Note that the docking profile may contain a docking score for each of the residues and an overall docking score for the peptide. The docking score for each of the residues is, for example, a computed value of electrostatic interaction energy between each of the blood plasma protein residues and the peptide, or a computed value of hydrophobic interaction energy therebetween. Moreover, the overall docking score of the peptide is, for example, a value computed from the docking scores of each of the residues.

Note that the prediction device 34 may also execute docking calculation between a preset region of the blood plasma protein and the peptide. For example, as illustrated in Fig. 15, a position is already known of the hydrophobic pocket H of the human serum albumin AL serving as the blood plasma protein, and so the docking calculation may be executed for a peripheral region to the hydrophobic pocket H as the preset region. There may, moreover, be plural such regions set separately.

Explanation follows regarding operation of the prediction device 510 of the fifth exemplary embodiment.

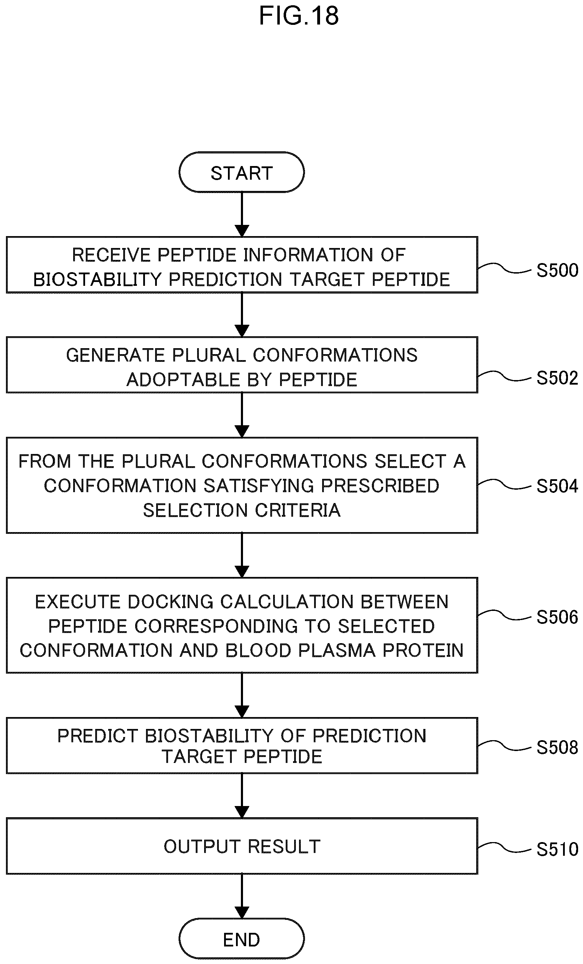

On receipt of an instruction signal indicating an instruction to start prediction processing, the prediction device 510 of the fifth exemplary embodiment executes the prediction processing routine illustrated in Fig. 18.

A step S500, the conformation generation section 32 acquires peptide information stored in the docking calculation data storage section 30 for the peptide that is the biostability prediction target.

At step S502, the conformation generation section 32 generates plural conformations adoptable by the biostability prediction target peptide based on the peptide information acquired at step S500. The conformation generation section 32 then temporarily stores information related to the plural conformations in the docking calculation data storage section 30.

At step S504, based on the prescribed selection criteria such as described above, the selection section 33 selects conformations to be subjected to docking calculation from out of the plural conformations generated at step S502. The conformation generation section 32 then temporarily stores the information related to the selected conformations in the docking calculation data storage section 30.

At step S506, for each of the conformations selected at the step S504, the prediction device 34 performs docking calculation between the biostability prediction target peptide corresponding to these respective conformations and the blood plasma protein. The prediction device 34 then temporarily stores the docking profile that is the docking calculation results thereof in the docking calculation data storage section 30.

At step S508, based on the docking profile obtained at step S506, the prediction device 34 predicts the biostability of the prediction target peptide by computing the prediction target biostability.

At step S510, the prediction device 34 outputs the predicted value of the peptide biostability computed at step S508 as a result.

As described above, the prediction device of the fifth exemplary embodiment generates plural conformations adoptable by the biostability prediction target peptide. The prediction device then, based on the prescribed selection criteria, selects conformations to be subjected to docking calculation from out of the plural generated conformations. The prediction device then predicts the biostability of the prediction target peptide by performing docking calculation between the prediction target peptide based on the selected conformation and the blood plasma protein. This thereby enables the biostability of the prediction target peptide to be predicted efficiently. Moreover, docking calculation is performed between a peptide and a blood plasma protein based on the selected conformation when predicting the biostability of the prediction target peptide, enabling the biostability of the peptide to be predicted with good accuracy by computing the biostability based on the computation results. A particular feature is the point that prediction is feasible even for novel peptides such as those that have been difficult to predict by a machine learning method due to there being insufficient precedent training data.

Sixth Exemplary Embodiment

Next, explanation follows regarding a sixth exemplary embodiment. A prediction device of the sixth exemplary embodiment differs from the first to fifth exemplary embodiments in respect that a predicted value for peptide biostability is computed by consolidating a predicted value for peptide biostability obtained by docking calculation with a predicted value for biostability obtained by a trained model built by machine learning. Note that similar portions in the configuration of the prediction device according to the sixth exemplary embodiment to those of any of the prediction devices of the first to fifth exemplary embodiments are allocated the same reference numerals, and explanation thereof is omitted.

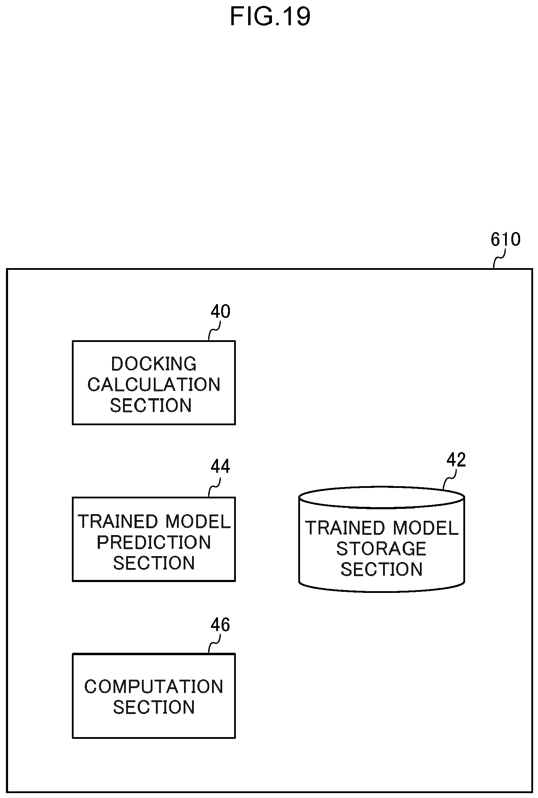

Fig. 19 is a block diagram illustrating an example of a configuration of a prediction device 610 according to the sixth exemplary embodiment. As illustrated in Fig. 19, in terms of functionality, the prediction device 610 includes a docking calculation section 40, a trained model storage section 42, a trained model prediction section 44, and a computation section 46.

The docking calculation section 40 generates a first biostability predicted value expressing biostability of a peptide by executing docking calculation between the biostability prediction target peptide and a blood plasma protein. For example, the docking calculation section 40 generates the first biostability predicted value expressing biostability of the peptide by a similar method to that of the prediction device of the fifth exemplary embodiment.

A trained model for outputting a predicted value for biostability of a peptide from a feature vector expressing a feature of a peptide is stored in the trained model storage section 42. For example, a trained model generated using any one of the prediction devices of the first to fourth exemplary embodiments is stored in the trained model storage section 42.

The trained model prediction section 44 extracts predictive feature vectors expressing features from the biostability prediction target peptide, and generates a second biostability predicted value expressing biostability of the peptide by inputting these predictive feature vectors into the trained model stored in the trained model storage section 42.

The computation section 46 computes a predicted value for biostability of the peptide by consolidating the first biostability predicted value generated by the docking calculation section 40 with the second biostability predicted value generated by the trained model prediction section 44. For example, the computation section 46 may compute a predicted value for biostability of the peptide by averaging the first biostability predicted value and the second biostability predicted value. Alternatively, the computation section 46 may compute the larger or smaller value out of the first biostability predicted value or the second biostability predicted value as being the predicted value for biostability of the peptide.

The computation section 46 outputs this predicted value for biostability of the peptide as a result.

As described above, the prediction device of the sixth exemplary embodiment generates the first biostability predicted value expressing peptide biostability by performing docking calculation between the prediction target peptide and the blood plasma protein. The prediction device also extracts predictive feature vectors expressing features from the peptide, and generates the second biostability predicted value expressing peptide biostability by inputting the predictive feature vectors into a pre-built trained model. The prediction device then computes a predicted value for biostability of the peptide by consolidating the generated first biostability predicted value with the generated second biostability predicted value. This enables a predicted value to be obtained that reflects both a predicted value obtained by docking calculation and a predicted value obtained using a trained model.

Seventh Exemplary Embodiment

Next, explanation follows regarding a seventh exemplary embodiment. A prediction device of the seventh exemplary embodiment differs from the first to sixth exemplary embodiments in respect that a trained model that employs a machine learning algorithm is built based on a docking profile obtained by docking calculation and from a feature value extracted from the peptide. Note that similar portions in the configuration of the prediction device according to the seventh exemplary embodiment to any of those in the prediction devices of the first to sixth exemplary embodiments are allocated the same reference numerals, and explanation thereof is omitted.

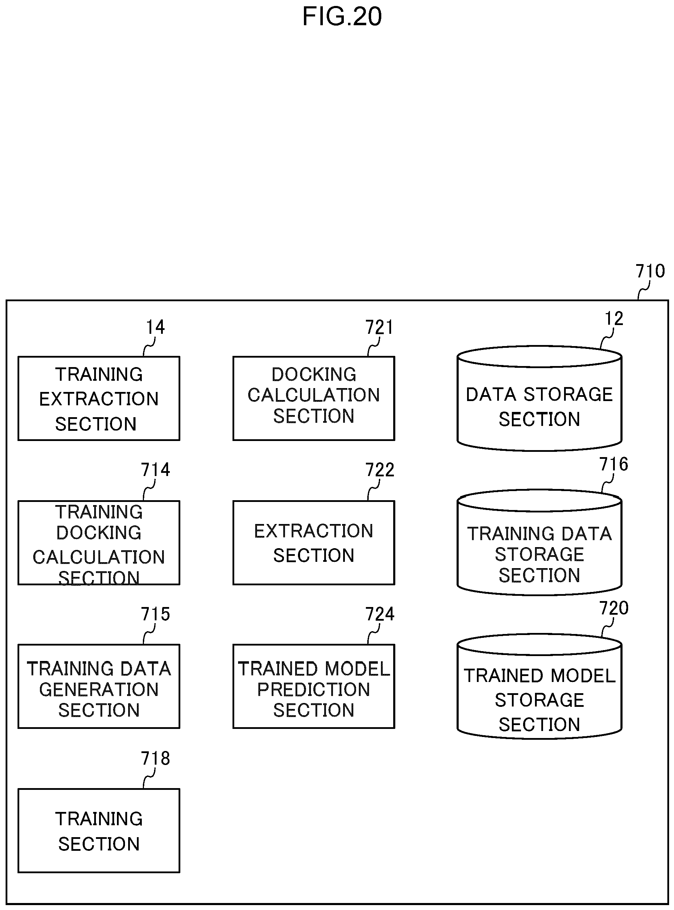

Fig. 20 is a block diagram illustrating an example of a configuration of a prediction device 710 according to a seventh exemplary embodiment. As illustrated in Fig. 20, the prediction device 710 includes, in terms of functionality, a data storage section 12, a training extraction section 14, a training docking calculation section 714, a training data generation section 715, a training data storage section 716, a training section 718, a trained model storage section 720, a docking calculation section 721, an extraction section 722, and a trained model prediction section 724.

For each of plural training peptides, the training extraction section 14 extracts feature values from peptide information of the training peptide by a method similar to one of the prediction devices of the first to the sixth exemplary embodiment.

The training docking calculation section 714 reads in peptide information from the data storage section 12 for plural training peptides. The training docking calculation section 714 then computes a training docking profile that is a docking profile for the training peptides by performing docking calculation between the training peptide information and the blood plasma protein for each of the plural training peptides.

The training data generation section 715 generates a training feature vector with elements that are the feature values extracted by the training extraction section 14 for each of the plural training peptides, and the training docking profile computed by the training docking calculation section 714. The training data generation section 715 then generates for each of the plural training peptides training data expressed by the training feature vectors paired with correct values of biostability. The training data generation section 715 then stores the plural generated training data in the training data storage section 716.

Plural of training data expressed by the training feature vectors paired with correct values of biostability are stored in the training data storage section 716. Note that the training feature vectors stored in the training data storage section 716 are training feature vectors including as elements the feature values extracted from the training peptides by the training extraction section 14 and the training docking profile computed by the training docking calculation section 714.

The training section 718 generates a trained model by executing a machine learning algorithm based on the plural training data stored in the training data storage section 716. The trained model is a model for outputting a predicted value of peptide biostability from a feature vector including a docking profile obtained by docking calculation of the peptide and a feature value extracted from the peptide.

The trained model generated by the training section 718 is stored in the trained model storage section 720.

The docking calculation section 721 computes the docking profile of the prediction target peptide by performing docking calculation between the biostability prediction target peptide and the blood plasma protein. Note that, for example, the docking calculation section 721 may perform a known docking calculation, or may perform docking calculation similar to that of the fifth exemplary embodiment.

The extraction section 722 extracts feature values from peptide information of the biostability prediction target peptide.

The trained model prediction section 724 generates a predictive feature vector having elements of the feature value extracted by the extraction section 722 and the docking profile computed by the docking calculation section 721. The trained model prediction section 724 then generates a predicted value of biostability for the prediction target peptide by inputting the predictive feature vector into the trained model stored in the trained model storage section 720.

This thereby enables the biostability of the prediction target peptide to be predicted with good accuracy by also including in the training data when generating the trained model the docking profile obtained by docking calculation.

A description now follows regarding operation of the prediction device 710 of the seventh exemplary embodiment.

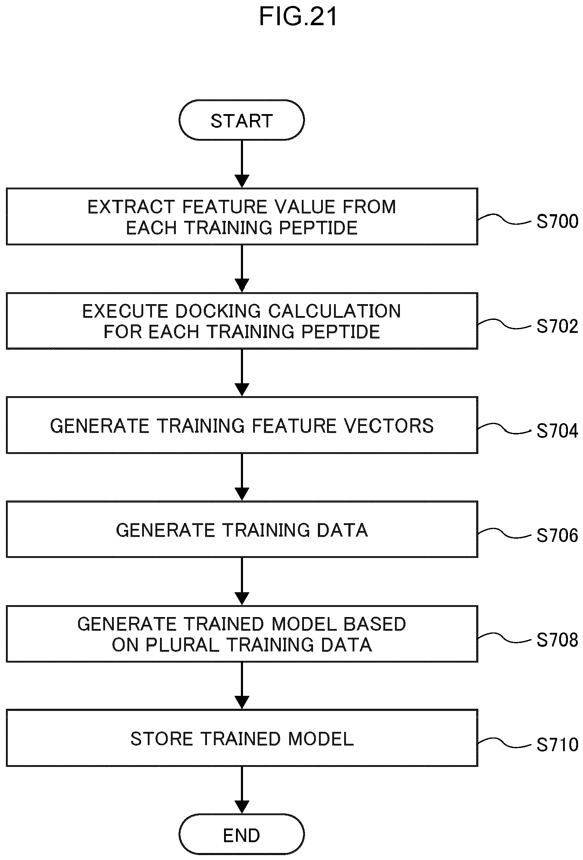

The prediction device 710 receives an instruction signal indicating an instruction to perform trained model generation processing, and executes a trained model generation processing routine illustrated in Fig. 21.

At step S700, the training extraction section 14 extracts feature values of the training peptides from the plural training peptide information stored in the data storage section 12.

At step S702, for each of the plural training peptide information stored in the data storage section 12, the training docking calculation section 714 computes a training docking profile of the training peptide by performing docking calculation between the training peptide information and the blood plasma protein.

At step S704, the training data generation section 715 generates a training feature vector having elements of the feature values extracted at step S700 for each of the plural training peptides and the training docking profiles computed at step S702.

At step S706, for each of the plural training peptides the training data generation section 715 generates training data expressed by the training feature vectors generated at step S704 paired with correct values of biostability. The training data generation section 715 then stores the plural generated training data in the training data storage section 716.

At step S708, based on the plural training data stored in the training data storage section 716, the training section 718 generates a trained model for outputting a predicted value for biostability of a peptide by executing a known supervised machine learning algorithm.

At step S710, the training section 718 stores the trained model generated at step S708 in the trained model storage section 720.

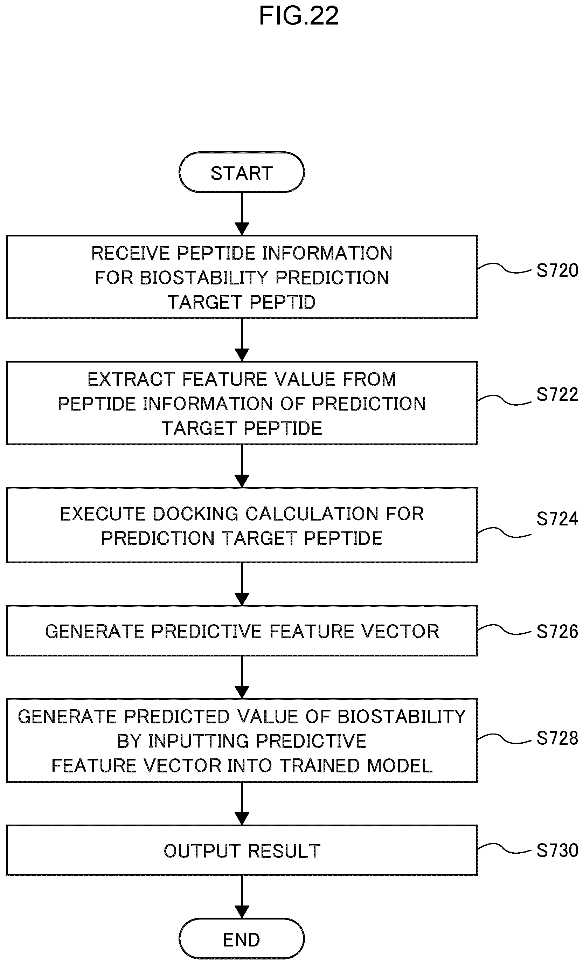

With the trained model has been stored in the trained model storage section 720, when the peptide information for the biostability prediction target is input to the prediction device 710, the prediction device 710 executes the prediction processing routine illustrated in Fig. 22.

At step S720, the extraction section 722 receives the peptide information for the biostability prediction target.

At step S722, the extraction section 722 extracts a feature value from the peptide information received at step S720.

At step S724, the docking calculation section 721 computes a docking profile of the prediction target peptide by performing a docking calculation between the peptide corresponding to the peptide information received at step S720 and the blood plasma protein.

At step S726, the trained model prediction section 724 generates a predictive feature vector having elements of the feature values extracted at step S722 and the docking profile computed at step S724.

At step S728, the trained model prediction section 724 generates a predicted value of biostability for the prediction target peptide by inputting the predictive feature vectors generated at step S724 into the trained model stored in the trained model storage section 720.

At step S730, the trained model prediction section 724 outputs the predicted value of biostability generated at step S728 as a result.

As described in detail above, the prediction device of the seventh exemplary embodiment computes a training docking profile that is a docking profile of the training peptides by performing docking calculation between the plural training peptides and the blood plasma protein. Based on the training data expressed by the training feature vector including the feature values extracted from the training peptides and the training docking profile for each of the plural training peptides paired with respective correct values of biostability of the training peptides, the prediction device generates a trained model, for outputting a predicted value of biostability of a peptide from a feature vector including a docking profile obtained by peptide docking calculation and feature values extracted from a peptide, by executing a machine learning algorithm. Including a docking profile obtained by docking calculation in the training data when generating the trained model in this manner enables a trained model to be obtained for predicting the biostability of the prediction target peptide with better accuracy.

Moreover, the prediction device of the seventh exemplary embodiment computes a docking profile including a docking score between a peptide and a blood plasma protein by performing docking calculation between the biostability prediction target peptide and the blood plasma protein. Note that the docking score is at least one out of a docking score for every residue or an overall docking score. The prediction device then generates a predicted value of biostability for the prediction target peptide by inputting the predictive feature vector including the computed docking profile into the trained model pre-generated using a machine learning algorithm. This thereby enables the biostability of the prediction target peptide to be predicted with good accuracy. Specifically, due to there being a lot of information included in the docking profile that is useful when predicting biostability, the biostability of the prediction target peptide can be predicted with better accuracy by utilizing this docking profile. More specifically, although 3D structural information of the blood plasma protein is not included in the feature values extracted from the peptide, the 3D structural information of the blood plasma protein is included in the docking profile, and this enables the biostability to also be predicted from a physical perspective. This enables the biostability of the prediction target peptide to be predicted with better accuracy by utilizing the docking profile.

Eighth Exemplary Embodiment

Explanation follows regarding an eighth exemplary embodiment. A prediction device of the eighth exemplary embodiment differs from the first to the seventh exemplary embodiments in the point that the prediction of biostability of the prediction target peptide utilizes a docking profile of residue docking calculation when docking calculation was performed between residues of the peptide and the blood plasma protein. Note that parts of the configuration of the prediction device according to the eighth exemplary embodiment similar to those of any of the prediction device of the first to the seventh exemplary embodiments are allocated the same reference numerals and description is omitted thereof.

The prediction device 710 of the seventh exemplary embodiment predicts the biostability by utilizing the docking profile that is the docking calculation result for the peptide as a whole. However, in such cases this means that a need arises to always perform docking calculation for each of the prediction target peptides. For example consider a case in which there is a peptide composed of residues [A, B, C, D, E, F], and there is a peptide composed of residues [A', B, C, D, E, F]. Even though the pair of peptides differ by merely a single residue, docking calculation for the peptide as a whole would still always executed for each of the peptides.

With regards to this point, for example as described above, a residue of the peptide is able to bind to a hydrophobic pocket in the blood plasma protein, and so the results of residue docking calculation for each of the residues is an important factor when predicting biostability.

Thus in the eighth exemplary embodiment, residue docking calculation is executed separately between each of the residues of plural types of peptide and the blood plasma protein. Then when predicting the biostability of the prediction target peptide, the prediction device of the eighth exemplary embodiment predicts the biostability of the peptide by utilizing the docking profiles of residue docking calculations that have been previously computed. A specific description follows thereof.

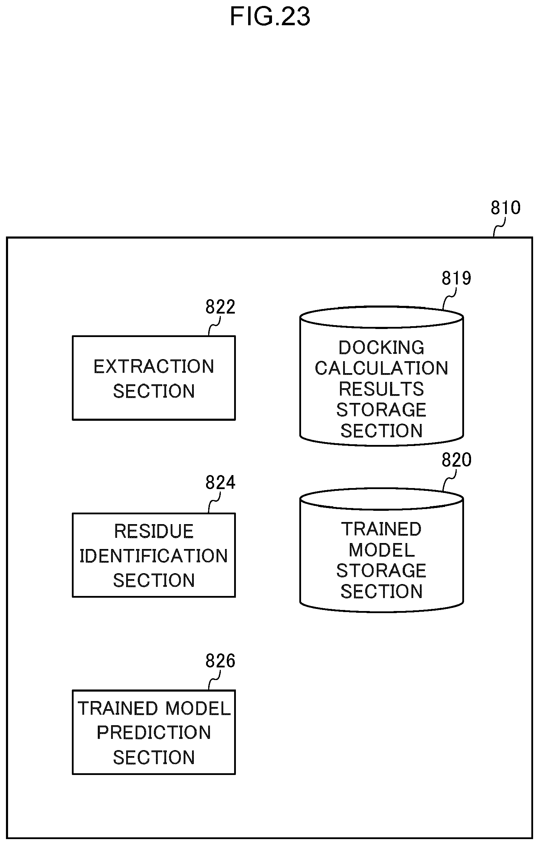

Fig. 23 is a block diagram illustrating an example of a configuration of a prediction device 810 according to the eighth exemplary embodiment. As illustrated in Fig. 23, the prediction device 810 includes, in terms of functionality, a docking calculation results storage section 819, a trained model storage section 820, an extraction section 822, a residue identification section 824, and a trained model prediction section 826.

Docking profiles for residues, which are the results of residue docking calculation for each residue for plural types of residue, are stored in the docking calculation results storage section 819. There are only limited types of residue, and so in the eighth exemplary embodiment the docking profiles of such residues are computed in advance and stored in the docking calculation results storage section 819.

A trained model, for predicting biostability of a peptide from a feature vector including a docking profile of residues of the peptide and a feature value extracted from the peptide, is stored in the trained model storage section 820. Note that the trained model is generated in advance using a machine learning algorithm based on training data expressed by the feature vectors of the training peptides paired with respective correct values of biostability of the training peptides. Note that the training feature vectors in such cases are feature vectors having elements of the docking profiles of the residues of the training peptides and the feature values extracted from the training peptides. Prediction of biostability employing the trained model is described later.

The extraction section 822 extracts a feature value from the peptide information of the biostability prediction target peptide. Note that there may be plural feature values present.

The residue identification section 824 identifies the types of residue in the biostability prediction target peptide. These types of residue are utilized when selecting docking profiles stored in the docking calculation results storage section 819.

The trained model prediction section 826 reads in the docking profiles corresponding to the types of residue identified by the residue identification section 824, and generates a predictive feature vector including the read docking profiles of the prediction target residues and the feature value extracted by the extraction section 822. The trained model prediction section 826 generates a predicted value of biostability of the prediction target peptide by inputting the predictive feature vector into the trained model stored in the trained model storage section 820.

Thus by executing the residue docking calculation in advance for residues in peptides and utilizing the docking profiles thereof in this manner, the biostability of the prediction target peptide can be predicted more efficiently. Moreover, due to residues being thought to be important factors of biostability, the biostability of the prediction target peptide can be predicted with good accuracy by utilizing these docking profiles.

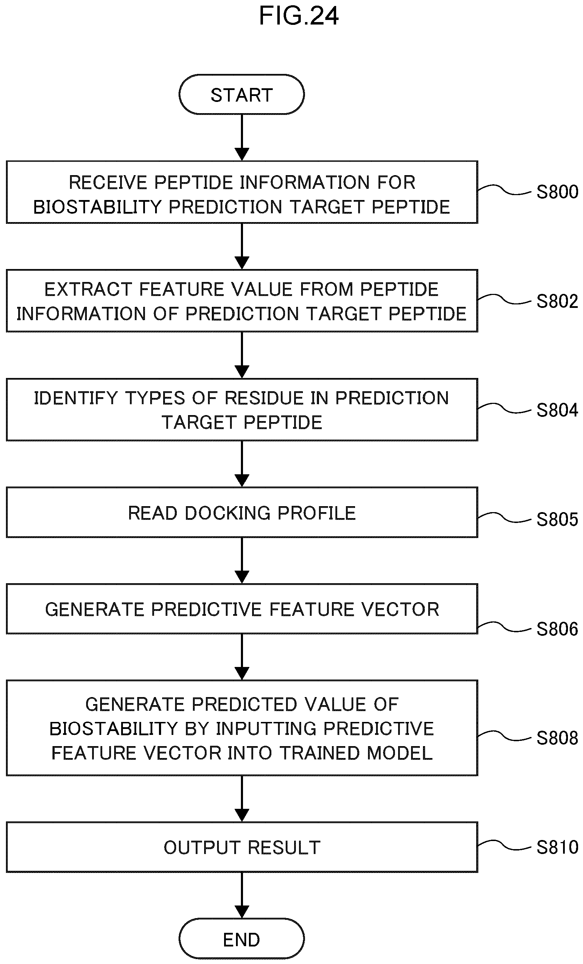

Next, description follows regarding operation of the prediction device 810 of the eighth exemplary embodiment. The trained model is stored in the trained model storage section 820, and when the peptide information of the biostability prediction target peptide has been input into the prediction device 810, the prediction device 810 executes a prediction processing routine illustrated in Fig. 24.

At step S800, the extraction section 822 receives the peptide information of the biostability prediction target peptide.

At step S802, the extraction section 822 extracts a feature value from the peptide information received at step S800.

At step S804, the residue identification section 824 identifies the types of residue in the peptide corresponding to the peptide information received at step S800.

At step S805, the trained model prediction section 826 reads the docking profiles corresponding to the types of residue identified at step S804 from the docking calculation results storage section 819.

At step S806, the trained model prediction section 826 generates a predictive feature vector having elements of the feature values extracted at step S802 and the docking profiles read at step S805.

At step S808, the trained model prediction section 826 generates a predicted value of biostability of the prediction target peptide by inputting the predictive feature vector generated at step S806 into the trained model stored in the trained model storage section 820.

At step S810, the trained model prediction section 826 outputs the predicted value of biostability generated at step S808 as a result.

As described in detail above, the prediction device of the eighth exemplary embodiment extracts residues from the biostability prediction target peptide. The prediction device then reads the docking profiles corresponding to the extracted residues from the storage section stored with docking profiles expressing the results of residue docking calculations between residues and a blood plasma protein for each of plural types of residue. The prediction device then predicts the biostability of the prediction target peptide by inputting a predictive feature vector including the read docking profiles of the prediction target residues into a trained model generated in advance by a machine learning algorithm. This thereby enables the biostability of the prediction target peptide to be predicted efficiently. Moreover, due to the types of residue being thought to be an important factor affecting biostability, the biostability of the prediction target peptide can be predicted with good accuracy by utilizing these docking profiles.

Note that the present disclosure is not limited to the exemplary embodiments described above, and various modifications and applications may be implemented within a range not departing from the spirit of the present disclosure.

For example, although an example has been described in the first exemplary embodiment of a case in which each feature vector is extracted for instances in which each of the plural residues contained in a cyclic peptide are at the start point of the cyclic sequence, these plural feature vectors are input into a trained model, and a representative value is obtained for the predicted values of biostability output from the trained model, there is no limitation thereto. For example, a single feature vector may be generated from each of the feature vectors for instances in which each of the plural residues contained in the cyclic peptide are at the start point of the cyclic sequence, this single feature vector input into a trained model, so as to obtain a predicted value of biostability. In such a case, for example, the single feature vector may be generated by taking a weighted average of the plural feature vectors. Moreover, for example, specific feature vectors may be selected from out of plural feature vectors, and a single feature vector generated by taking a weighted average of the plural feature vectors that have been selected. Moreover, even when generating the trained model, a single training feature vector may be generated from each of the training feature vectors for instances in which each of the plural residues contained in the cyclic peptide are at the start point of the cyclic sequence, and then this training feature vector employed so as to generate the trained model.

Moreover, although an example was described above in the seventh exemplary embodiment of a case in which the training feature vector and the predictive feature vector are each a vector having elements of the feature value extracted from the peptide and a docking profile, there is no limitation thereto. For example, the training feature vector and the predictive feature vector may be a vector having elements of the docking profile alone. Moreover, this docking profile may include only docking scores obtained for each of the residues on the blood plasma protein side, or may further include an overall docking score expressing a total of the docking scores for each of the residues.

Moreover, although an example was described above in the seventh exemplary embodiment of a case in which the docking profiles of each of the residues was computed in advance, there is no limitation thereto. For example, from the residues the docking calculation may be executed in advance for those of the side chain portions alone and excluding the main chain structure, and the docking profile for each of the side chains stored in advance in the docking calculation results storage section 819.

Moreover, although in the above exemplary embodiment examples have been described of cases in which the trained model is generated based on training data, there is no limitation thereto. For example, the trained model of the present exemplary embodiment may be generated as a distillation model based on other trained models.

Moreover, although embodiments have been described above in which a program according to the present disclosure is pre-stored (installed) in a storage section (not illustrated in the drawings), the program according to the present disclosure may be provided in a format recorded on a recording medium such as a CD-ROM, a DVD-ROM, a micro SD card, or the like.

Note that although in the above exemplary embodiments a CPU reads in software (a program) and executes processing thereof, various processors other than a CPU may be employed for execution. Processors in such cases include programmable logic devices (PLD) that allow circuit configuration to be modified post-manufacture, such as a field-programmable gate array (FPGA), and dedicated electric circuits, these being processors including a circuit configuration custom-designed to execute specific processing, such as an application specific integrated circuit (ASIC). The processing may be executed by any one of these various types of processor, or may be executed by a combination of two or more of the same type or different types of processor (such as plural FPGAs, or a combination of a CPU and an FPGA). The hardware structure of these various types of processors is more specifically an electric circuit combining circuit elements such as semiconductor elements.

Moreover, the respective processing of the exemplary embodiments may be executed by the processing being executed by a program in a configuration of a computer, a server, or the like including a generic computation processing device, a storage device, and the like. Such a program may be stored in a storage device or recorded on a recording medium such as a magnetic disc, an optical disc, or semiconductor memory, or provided over a network. Obviously, other configuration elements also do not need to be implemented using a single computer or server, and may be distributed across and implemented by plural computers that are connected together over a network.

The disclosures of Japanese Patent Application No. 2021-035648, filed on March 5, 2021, are incorporated herein by reference in their entirety. All publications, patent applications, and technical standards mentioned in this specification are herein incorporated by reference to the same extent as if each individual publication, patent application, or technical standard was specifically and individually indicated to be incorporated by reference.

## Claims

1. A prediction device comprising:

   an extraction section configured to extract a predictive feature vector expressing a feature from a peptide that is a target for biostability prediction;
   an adjustment section configured to adjust a length of the predictive feature vector extracted by the extraction section to a prescribed length; and
   a generation section configured to generate a predicted value of biostability for the prediction target peptide by inputting the predictive feature vector adjusted in length by the adjustment section into a trained model pre-trained to output a predicted value of peptide biostability from a feature vector expressing a feature of a peptide.

2. The prediction device of claim 1, wherein the adjustment section is configured to adjust the length of the predictive feature vector by a padding method or by conversion using a linear interpolation method.

3. A trained model generation device comprising:

   a training extraction section configured to extract a training feature vector expressing a feature from each of a plurality of training peptides;
   a training adjustment section configured to adjust a length of each training feature vector for each of the plurality of training peptides extracted by the training extraction section to a prescribed length; and
   a training section configured to generate a trained model for outputting a predicted value for peptide biostability from a feature vector expressing a feature of a peptide by executing a machine learning algorithm based on training data that is the training feature vectors adjusted in length by the training adjustment section paired with correct values of biostability for the training peptides.

4. A prediction device comprising:

   an extraction section configured to extract each predictive feature vector expressing a feature from a cyclic peptide that is a target for biostability prediction for instances in which each of a plurality of residues contained in the cyclic peptide is at a start point of a cyclic sequence; and

a generation section configured to generate a predicted value for biostability of the prediction target cyclic peptide by inputting a plurality of predictive feature vectors extracted by the extraction section into a trained model pre-trained to output a predicted value of peptide biostability from a feature vector expressing a feature of a cyclic peptide.

5. The prediction device of claim 4, wherein the generation section is configured to input each of the plurality of predictive feature vectors into the trained model and to generate a representative value of a predicted value for biostability of the prediction target cyclic peptide for each of a plurality of feature vectors output from the trained model.

6. A trained model generation device comprising:

a training extraction section configured to extract a training feature vector expressing a feature from out of a plurality of training cyclic peptides for instances in which each of a plurality of residues contained in the respective training cyclic peptide is at a start point of a cyclic sequence; and

a training section configured to generate a trained model for outputting a predicted value of biostability of a cyclic peptide from a feature vector expressing a feature of a cyclic peptide by executing a machine learning algorithm based on training data that is a plurality of training feature vectors extracted by the training extraction section for each of a plurality of training cyclic peptides paired with a correct value of biostability for the respective training cyclic peptide.

7. A trained model generation device comprising:

a training extraction section configured to extract a first training feature vector expressing a feature from each of a plurality of training cyclic peptides;

a training data generation section configured to generate a plurality of second training feature vectors for each of the first training feature vectors extracted by the training extraction section by cyclically shifting elements of the first training feature vector, and to generate training data expressed by the first training feature vector and the plurality of second training feature vectors paired with a correct value for biostability of the respective training cyclic peptide; and

a training section configured to generate a trained model for outputting a predicted value of biostability of a cyclic peptide from a feature vector expressing a feature of a cyclic peptide by executing a machine learning algorithm based on a plurality of training data generated by the training data generation section.

8. A prediction device comprising:

an extraction section configured to extract a predictive feature vector expressing a feature from a cyclic peptide that is a target for biostability prediction; and

a generation section configured to generate a predicted value of biostability for the prediction target peptide by inputting the predictive feature vector extracted by the extraction section into the trained model generated by the trained model generation device of claim 7.

9. A trained model generation device comprising:
a training section configured to generate a trained convolutional neural network model for outputting a predicted value of biostability of a cyclic peptide from a feature vector expressing a feature of a cyclic peptide by, based on training data expressed by a training feature vector expressing a feature extracted from each of a plurality of training cyclic peptides paired with a correct value of biostability for the plurality of respective training cyclic peptides, executing a machine learning algorithm employing a convolutional neural network model including a both-end-adjacency layer in which elements at both ends of the training feature vector are placed adjacent to one another.

10. A prediction device comprising:

an extraction section configured to extract a predictive feature vector expressing a feature from a cyclic peptide that is a target for biostability prediction; and

a generation section configured to generate a predicted value of biostability of the prediction target peptide by inputting the predictive feature vector extracted by the extraction section into a trained convolutional neural network model including a both-end-adjacency layer in which elements at both ends of a feature vector expressing a feature of a cyclic peptide are placed adjacent to one another, the trained convolutional neural network model being configured to output a predicted value of biostability of a peptide from the feature vector.

11. A prediction device comprising:

a conformation generation section configured to generate a plurality of conformations adoptable by a peptide that is a target for biostability prediction;
a selection section configured to select a conformation to be subjected to docking calculation from out of the plurality of conformations generated by the conformation generation section based on a prescribed selection criteria; and
a prediction section configured to predict an biostability of the prediction target peptide by performing docking calculation between the prediction target peptide corresponding to the conformation selected by the selection section and a blood plasma protein.

12. The prediction device of claim 11, wherein the selection section selects the conformation to be subjected to docking calculation from the plurality of conformations based on at least one factor from out of:

a straightness of a side chain of the prediction target peptide when adopting the conformation;
a degree of linearity of a side chain of the prediction target peptide when adopting the conformation;
a structure of a root portion of a side chain of the prediction target peptide when adopting the conformation;
a three dimensional shape of a vicinity of a leading end portion of a side chain of the prediction target peptide when adopting the conformation; or
a physical condition representing presence or absence of a charged atom contained in a side chain of the prediction target peptide when adopting the conformation.

13. A prediction device comprising:

a docking calculation section configured to compute a predicted value of a first biostability expressing an biostability of a peptide that is a target for biostability prediction by performing docking calculation between the peptide and a blood plasma protein;
a trained model prediction section configured to generate a predicted value of a second biostability expressing an biostability of the peptide by inputting a feature vector extracted from the prediction target peptide into a trained model generated in advance by a machine learning algorithm; and
a computation section configured to compute an biostability of the peptide by consolidating the first biostability predicted value generated by the docking calculation section with the second biostability predicted value generated by the trained model prediction section.

14. A prediction device comprising:

a docking calculation section configured to compute a docking profile including a docking score between a peptide that is a target for biostability prediction and a blood plasma protein by performing docking calculation between the peptide and the blood plasma protein; and
a trained model prediction section configured to generate a predicted value of biostability of the prediction target peptide by inputting a predictive feature vector including the docking profile computed by the docking calculation section into a trained model generated in advance by a machine learning algorithm.

15. The prediction device of claim 14, wherein the docking profile includes at least one of:

a docking score between the peptide and each residue in a pocket of the blood plasma protein; or
an overall docking score between the peptide and the blood plasma protein.

16. The prediction device of claim 14 or claim 15 wherein:

the prediction device further comprises an extraction section configured to extract a feature value expressing a feature from the prediction target peptide; and
the trained model prediction section generates a predicted value of biostability of the prediction target peptide by inputting the predictive feature vector including the docking profile and the feature value extracted by the extraction section into the trained model.

**17.** A trained model generation device comprising:

a training docking calculation section configured to compute a training docking profile that is a docking profile including a docking score of a plurality of training peptides by performing docking calculations between the respective training peptides and a blood plasma protein; and
a training section configured to generate a trained model for outputting a predicted value of biostability of a peptide from a feature vector including a docking profile obtained by docking calculation of a peptide by executing a machine learning algorithm on each of a plurality of the training peptides based on training data expressed by a training feature vector including the training docking profile computed by the training docking calculation section paired with a correct value of biostability of the respective training peptide.

**18.** A prediction device comprising:

an extraction section configured to extract a residue from a peptide that is a target for biostability prediction; and
a trained model prediction section configured to predict biostability of the biostability prediction target peptide by, for each of a plurality of types of residue, reading a docking profile corresponding to the residue extracted by the extraction section from a storage section stored with a docking profile expressing a result of docking calculation between the residue and a blood plasma protein, and inputting a feature vector including a read docking profile of the prediction target residue into a trained model generated in advance by a machine learning algorithm.

**19.** A prediction method comprising:
by a computer:

extracting a predictive feature vector expressing a feature from a peptide that is a target for biostability prediction;
adjusting a length of the extracted predictive feature vector to a prescribed length; and
generating a predicted value of biostability for the prediction target peptide by inputting the predictive feature vector adjusted in length into a trained model pre-trained to output a predicted value of peptide biostability from a feature vector expressing a feature of a peptide.

**20.** A trained model generation method comprising:
by a computer:

extracting a training feature vector expressing a feature from each of a plurality of training peptides;
adjusting a length of each training feature vector for each of the plurality of extracted training peptides to a prescribed length; and
generating a trained model, for outputting a predicted value for peptide biostability of a peptide from a feature vector expressing a feature of a peptide by executing a machine learning algorithm based on training data that is the length-adjusted training feature vectors paired with correct values of biostability for the respective training peptides.

**21.** A prediction method comprising:
by a computer:

extracting a predictive feature vector expressing a feature from a cyclic peptide that is a target for biostability prediction for instances in which each of a plurality of residues contained in the cyclic peptide is at a start point of a cyclic sequence; and
generating a predicted value of biostability of the prediction target cyclic peptide by inputting a plurality of the extracted predictive feature vectors into a trained model pre-trained to output a predicted value of biostability of a peptide from a feature vector expressing a feature of a cyclic peptide.

**22.** A trained model generation method comprising:
by a computer:

extracting a training feature vector expressing a feature from each of a plurality of training cyclic peptides for instances in which each of a plurality of residues contained in the training cyclic peptide is at a start point of a cyclic sequence; and
generating a trained model for outputting a predicted value of biostability of a cyclic peptide from a feature vector expressing a feature of a cyclic peptide by executing a machine learning algorithm based on training data expressed by a plurality of training feature vectors extracted for each of a plurality of training cyclic peptides paired with correct values of biostability for the training cyclic peptides.

**23.** A trained model generation method comprising:
by a computer:

extracting a first training feature vector expressing a feature from each of a plurality of training cyclic peptides;
generating a plurality of second training feature vectors for each of the extracted first training feature vectors by cyclically shifting elements of the first training feature vector, and generating training data expressed by the first training feature vector and the plurality of second training feature vectors paired with a correct value for biostability of the respective training cyclic peptide; and
generating a trained model for outputting a predicted value of biostability of a cyclic peptide from a feature vector expressing a feature of the cyclic peptide by executing a machine learning algorithm based on a plurality of the generated training data.

**24.** A prediction method comprising:
by a computer:

extracting a predictive feature vector expressing a feature from a cyclic peptide that is a target for biostability prediction; and
generating a predicted value for biostability of the prediction target peptide by inputting the extracted predictive feature vector into a trained model generated by the trained model generation method of claim 23.

**25.** A trained model generation method comprising:
by a computer:
generating a trained convolutional neural network model for outputting a predicted value of biostability of a cyclic peptide from a feature vector expressing a feature of a cyclic peptide by, based on training data expressed by a training feature vector expressing a feature extracted from each of a plurality of training cyclic peptides paired with a correct value of biostability for the plurality of respective training cyclic peptides, executing a machine learning algorithm using a convolutional neural network model including a both-end-adjacency layer in which elements at both ends of the training feature vector are placed adjacent to one another.

**26.** A prediction method comprising:
by a computer:

extracting a predictive feature vector expressing a feature from a cyclic peptide that is a target for biostability prediction; and
generating a predicted value of biostability of the prediction target cyclic peptide by inputting the extracted predictive feature vector into a trained convolutional neural network model that is a trained convolutional neural network model including a both-end-adjacency layer in which elements at both ends of a feature vector expressing a feature of a cyclic peptide are placed adjacent to one another and that is configured to output a predicted value of biostability of a peptide from the feature vector.

**27.** A prediction method comprising:
by a computer:

generating a plurality of conformations adoptable by a peptide that is a target for biostability prediction;
selecting a conformation to be subjected to docking calculation from the plurality of generated conformations based on a prescribed selection criteria; and
predicting the biostability of the prediction target peptide by performing docking calculation between a prediction target peptide corresponding to the selected conformation and a blood plasma protein.

**28.** A prediction method comprising:
by a computer:

computing a predicted value of a first biostability expressing an biostability of a peptide that is a target for biostability prediction by performing docking calculation between the biostability prediction target peptide and a blood plasma protein;

generating a predicted value of a second biostability predicted value expressing biostability of the peptide by inputting a feature vector extracted from the prediction target peptide into a trained model generated in advance by a machine learning algorithm; and

computing biostability of the peptide by consolidating the generated first biostability predicted value with the second biostability predicted value.

29. A prediction method comprising:

by a computer:

computing a docking profile including a docking score between a peptide that is a target for biostability prediction and a blood plasma protein by performing docking calculation between the peptide and the blood plasma protein; and

generating a predicted value of biostability for the prediction target peptide by inputting a predictive feature vector including the computed docking profile into a trained model generated in advance by a machine learning algorithm.

30. A trained model generation method comprising:

by a computer:

computing a training docking profile that is a docking profile including a docking score of a training peptide by performing docking calculation between a plurality of training peptides and a blood plasma protein; and

generating a trained model for outputting a predicted value of biostability of a peptide from a feature vector including a docking profile obtained by performing docking calculation of the peptide by executing a machine learning algorithm based on training data expressed by a training feature vector including a computed training docking profile for each of a plurality of training peptides paired with a correct value of biostability for the respective training peptides.

31. A prediction method comprising:

by a computer:

extracting a residue from a peptide that is a target for biostability prediction; and

predicting biostability of the prediction target peptide by reading a docking profile corresponding to the extracted residue from a storage section stored with docking profiles expressing results of docking calculations between a residue and a blood plasma protein for each of a plurality of types of residue, and by inputting a feature vector including the read docking profile of the prediction target residue into a trained model generated in advance by a machine learning algorithm.

32. A recording medium storing a prediction program executable by a computer to perform processing of the prediction method of any one of claim 19, claim 21, claim 24, claim 26, claim 27, claim 28, claim 29, or claim 31.

33. A recording medium storing a trained model generation program executable by a computer to perform processing of the trained model generation method of any one of claim 20, claim 22, claim 23, claim 25, or claim 30.

# FIG.1

TRAINING
EXTRACTION
SECTION
14

EXTRACTION
SECTION
22

DATA STORAGE
SECTION
12

TRAINING
SECTION
18

GENERATION
SECTION
24

TRAINING DATA
STORAGE
SECTION
16

TRAINED MODEL
STORAGE
SECTION
20

10

FIG.2

| DATA ID | TRAINING PEPTIDE INFORMATION | CORRECT VALUE FOR BIOSTABILITY OF TRAINING PEPTIDE |
|---|---|---|
| 00001 | Pe1 | Te1 |
| ... | ... | ... |
| ... | ... | ... |

# FIG.3A

# FIG.3B

RESIDUE 2

RESIDUE 3

RESIDUE 1

RESIDUE 4

RESIDUE 8

RESIDUE 5

RESIDUE 7

RESIDUE 6

# FIG.4A

| DATA ID | TRAINING FEATURE VECTOR | | CORRECT VALUE FOR BIOSTABILITY OF TRAINING PEPTIDE |
|---------|----------------|---|---------------------------------|
| 00001 | Fv1 | | Te1 |
| | Fv2 | | |
| | ... | | |
| | ... | | |
| ... | ... | | ... |
| ... | ... | | ... |

# FIG.4B

FEATURE VECTOR

TRAINED MODEL

PREDICTED VALUE FOR BIOSTABILITY 0.80

FIG.5

# FIG.6

START

EXTRACT RESPECTIVE TRAINING FEATURE
VECTORS FOR INSTANCES IN WHICH EACH OF
PLURAL RESIDUES CONTAINED IN A
TRAINING CYCLIC PEPTIDE IS AT A START
POINT OF THE CYCLIC SEQUENCE ～ S100

GENERATE TRAINING DATA ～ S102

GENERATE A TRAINED MODEL BASED
ON PLURAL TRAINING DATA ～ S104

STORE TRAINED MODEL ～ S106

END

# FIG.7

```
START
```

RECEIVE PEPTIDE INFORMATION FOR
A CYCLIC PEPTIDE THAT IS THE TARGET
FOR BIOSTABILITY PREDICTION — S200

EXTRACT RESPECTIVE FEATURE VECTORS
FOR INSTANCES IN WHICH EACH OF
PLURAL RESIDUES CONTAINED IN THE CYCLIC
PEPTIDE IS AT A START POINT OF THE
CYCLIC SEQUENCE — S202

GENERATE A PREDICTED VALUE OF
BIOSTABILITY BY INPUTTING THE
PLURAL FEATURE VECTORS INTO
THE TRAINED MODEL — S204

GENERATE A REPRESENTATIVE VALUE
FOR BIOSTABILITY — S206

OUTPUT A RESULT — S208

```
END
```

# FIG.8

210

| | | |
|---|---|---|
| 14 | 22 | 12 |
| TRAINING EXTRACTION SECTION | EXTRACTION SECTION | DATA STORAGE SECTION |
| 15 | 23 | 16 |
| TRAINING ADJUSTMENT SECTION | ADJUSTMENT SECTION | TRAINING DATA STORAGE SECTION |
| 18 | 24 | 20 |
| TRAINING SECTION | GENERATION SECTION | TRAINED MODEL STORAGE SECTION |

# FIG.9

START

EXTRACT TRAINING FEATURE VECTORS
FROM PLURAL TRAINING PEPTIDE INFORMATION — S300

PERFORM ADJUSTMENT SUCH THAT LENGTHS
OF RESPECTIVE PLURAL TRAINING FEATURE
VECTORS BECOME PRESCRIBED LENGTH — S302

GENERATE TRAINING DATA — S304

GENERATE TRAINED MODEL BASED
ON THE PLURAL TRAINING DATA — S306

STORE TRAINED MODEL — S308

END

# FIG.10

```
           ( START )
               │
               ▼
┌───────────────────────────────┐
│ RECEIVE PEPTIDE INFORMATION   │
│ FOR A TARGET FOR BIOSTABILITY │─── S400
│ PREDICTION                    │
└───────────────────────────────┘
               │
               ▼
┌───────────────────────────────┐
│ EXTRACT FEATURE VECTORS FROM  │─── S402
│ PEPTIDE INFORMATION           │
└───────────────────────────────┘
               │
               ▼
┌───────────────────────────────┐
│ PERFORM ADJUSTMENT SUCH THAT  │
│ LENGTHS OF FEATURE VECTORS    │─── S404
│ BECOME PRESCRIBED LENGTH      │
└───────────────────────────────┘
               │
               ▼
┌───────────────────────────────┐
│ GENERATE A PREDICTED VALUE OF │
│ BIOSTABILITY BY INPUTTING     │─── S406
│ LENGTH-ADJUSTED FEATURE       │
│ VECTOR INTO TRAINED MODEL     │
└───────────────────────────────┘
               │
               ▼
┌───────────────────────────────┐
│       OUTPUT RESULT           │─── S408
└───────────────────────────────┘
               │
               ▼
            ( END )
```

# FIG.11

310

| | | |
|---|---|---|
| 14 | 22 | 12 |
| TRAINING EXTRACTION SECTION | EXTRACTION SECTION | DATA STORAGE SECTION |
| 315 | 24 | 16 |
| TRAINING DATA GENERATION SECTION | GENERATION SECTION | TRAINING DATA STORAGE SECTION |
| 18 | | 20 |
| TRAINING SECTION | | TRAINED MODEL STORAGE SECTION |

FIG.12

| | 1 | 2 | 3 | 4 | 5 | 6 |
|---|---|---|---|---|---|---|
| FIRST TRAINING FEATURE VECTOR | A | B | C | D | E | F |
| SECOND TRAINING FEATURE VECTORS | B | C | D | E | F | A |
| | C | D | E | F | A | B |
| | D | E | F | A | B | C |
| | E | F | A | B | C | D |
| | F | A | B | C | D | E |

# FIG.13

CNN1

| 0 | A | B | C | 0 |
|---|---|---|---|---|

I

| 0AB | ABC | BC0 |
|-----|-----|-----|

Cv

# FIG.14

CNN2

| 0 | A | B | C | 0 | ~ I |

| 0 | C | A | B | C | A | 0 | ~ I' |

| 0CA | CAB | ABC | BCA | CA0 | ~ Cv |

# FIG.15

# FIG.16

# FIG.17

510

32

CONFORMATION
GENERATION
SECTION

30

DOCKING
CALCULATION
DATA STORAGE
SECTION

33

SELECTION
SECTION

34

PREDICTION
DEVICE

# FIG.18

START

RECEIVE PEPTIDE INFORMATION OF BIOSTABILITY PREDICTION TARGET PEPTIDE — S500

GENERATE PLURAL CONFORMATIONS ADOPTABLE BY PEPTIDE — S502

FROM THE PLURAL CONFORMATIONS SELECT A CONFORMATION SATISFYING PRESCRIBED SELECTION CRITERIA — S504

EXECUTE DOCKING CALCULATION BETWEEN PEPTIDE CORRESPONDING TO SELECTED CONFORMATION AND BLOOD PLASMA PROTEIN — S506

PREDICT BIOSTABILITY OF PREDICTION TARGET PEPTIDE — S508

OUTPUT RESULT — S510

END

# FIG.19

610

**40**

DOCKING
CALCULATION
SECTION

**44**

TRAINED MODEL
PREDICTION
SECTION

**42**

TRAINED MODEL
STORAGE
SECTION

**46**

COMPUTATION
SECTION

# FIG.20

710

| | | |
|---|---|---|
| 14 TRAINING EXTRACTION SECTION | 721 DOCKING CALCULATION SECTION | 12 DATA STORAGE SECTION |
| 714 TRAINING DOCKING CALCULATION SECTION | 722 EXTRACTION SECTION | 716 TRAINING DATA STORAGE SECTION |
| 715 TRAINING DATA GENERATION SECTION | 724 TRAINED MODEL PREDICTION SECTION | 720 TRAINED MODEL STORAGE SECTION |
| 718 TRAINING SECTION | | |

# FIG.21

START

EXTRACT FEATURE VALUE FROM
EACH TRAINING PEPTIDE — S700

EXECUTE DOCKING CALCULATION
FOR EACH TRAINING PEPTIDE — S702

GENERATE TRAINING FEATURE VECTORS — S704

GENERATE TRAINING DATA — S706

GENERATE TRAINED MODEL BASED
ON PLURAL TRAINING DATA — S708

STORE TRAINED MODEL — S710

END

# FIG.22

START

RECEIVE PEPTIDE INFORMATION
FOR BIOSTABILITY PREDICTION
TARGET PEPTID — S720

EXTRACT FEATURE VALUE FROM
PEPTIDE INFORMATION OF PREDICTION
TARGET PEPTIDE — S722

EXECUTE DOCKING CALCULATION FOR
PREDICTION TARGET PEPTIDE — S724

GENERATE PREDICTIVE FEATURE VECTOR — S726

GENERATE PREDICTED VALUE OF BIOSTABILITY
BY INPUTTING PREDICTIVE
FEATURE VECTOR INTO TRAINED MODEL — S728

OUTPUT RESULT — S730

END

# FIG.23

810

822

EXTRACTION
SECTION

819

DOCKING
CALCULATION
RESULTS
STORAGE
SECTION

824

RESIDUE
IDENTIFICATION
SECTION

820

TRAINED
MODEL
STORAGE
SECTION

826

TRAINED MODEL
PREDICTION
SECTION

# FIG.24

START

RECEIVE PEPTIDE INFORMATION FOR
BIOSTABILITY PREDICTION TARGET PEPTIDE — S800

EXTRACT FEATURE VALUE FROM PEPTIDE
INFORMATION OF PREDICTION TARGET PEPTIDE — S802

IDENTIFY TYPES OF RESIDUE IN PREDICTION
TARGET PEPTIDE — S804

READ DOCKING PROFILE — S805

GENERATE PREDICTIVE FEATURE VECTOR — S806

GENERATE PREDICTED VALUE OF
BIOSTABILITY BY INPUTTING PREDICTIVE
FEATURE VECTOR INTO TRAINED MODEL — S808

OUTPUT RESULT — S810

END

Europäisches
Patentamt

European
Patent Office

Office européen
des brevets

## PARTIAL EUROPEAN SEARCH REPORT

Application Number

under Rule 62a and/or 63 of the European Patent Convention.
This report shall be considered, for the purposes of
subsequent proceedings, as the European search report

EP 22 15 9146

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KURCINSKI MATEUSZ ET AL: "Flexible docking of peptides to proteins using CABS-dock", PROTEIN SCIENCE, [Online] vol. 29, no. 1, 1 January 2020 (2020-01-01), pages 211-222, XP055977369, US ISSN: 0961-8368, DOI: 10.1002/pro.3771 Retrieved from the Internet: URL:https://www.ncbi.nlm.nih.gov/pmc/artic les/PMC6933849/pdf/PRO-29-211.pdf> [retrieved on 2022-11-02] * the whole document * * in particular * * sections 3 and 4; figures 3-5 * ----- -/-- | 11,12, 27,32 | INV. G16B15/30 G16B40/20 G16C20/30 G16C20/50 G16C20/70 |

TECHNICAL FIELDS
SEARCHED (IPC)

G16B
G16C

## INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:

see sheet C

| Place of search | Date of completion of the search | Examiner |
|---|---|---|
| Munich | 2 November 2022 | Rákossy, Z |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..................................................................................
& : member of the same patent family, corresponding document

EPO FORM 1503 03.82 (P04E07)

Europäisches
Patentamt

European
Patent Office

Office européen
des brevets

**PARTIAL EUROPEAN SEARCH REPORT**

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| X | LIAO JUN-MIN ET AL: "A fragment-based docking simulation for investigating peptide-protein bindings", PHYSICAL CHEMISTRY CHEMICAL PHYSICS, [Online] vol. 19, no. 16, 24 March 2017 (2017-03-24), pages 10436-10442, XP055977375, ISSN: 1463-9076, DOI: 10.1039/C6CP07136H Retrieved from the Internet: URL:https://pubs.rsc.org/en/content/articl epdf/2017/cp/c6cp07136h> [retrieved on 2022-11-02] * the whole document * * in particular * * results and methods sections * ----- | 11,12, 27,32 | |
| X | US 2019/272887 A1 (FEINBERG EVAN NATHANIEL [US] ET AL) 5 September 2019 (2019-09-05) * the whole document * * in particular * * paragraph [0005] – paragraph [0023]; figure 6 * ----- | 11,12, 27,32 | |
| X | US 2018/341754 A1 (FAN JIE [US] ET AL) 29 November 2018 (2018-11-29) * the whole document * * in particular * * paragraph [0014] – paragraph [0027] * * paragraph [0062] – paragraph [0133] * ----- -/-- | 11,12, 27,32 | |

TECHNICAL FIELDS SEARCHED (IPC)

EPO FORM 1503 03.82 (P04C10)

Europäisches
Patentamt

European
Patent Office

Office européen
des brevets

**PARTIAL EUROPEAN SEARCH REPORT**

Application Number

EP 22 15 9146

## DOCUMENTS CONSIDERED TO BE RELEVANT

| Category | Citation of document with indication, where appropriate, of relevant passages | Relevant to claim |
|---|---|---|
| X | CIEMNY MACIEJ ET AL: "Protein-peptide docking: opportunities and challenges", DRUG DISCOVERY TODAY, [Online] vol. 23, no. 8, 4 May 2018 (2018-05-04), pages 1530-1537, XP055977383, AMSTERDAM, NL ISSN: 1359-6446, DOI: 10.1016/j.drudis.2018.05.006 [retrieved on 2022-11-02] * the whole document * * in particular * * page 1534, left-hand column; table 1 * ----- | 11,12, 27,32 |

**CLASSIFICATION OF THE APPLICATION (IPC)**

**TECHNICAL FIELDS SEARCHED (IPC)**

EPO FORM 1503 03.82 (P04C10)

page 3 of 3

Europäisches
Patentamt

European
Patent Office

Office européen
des brevets

**INCOMPLETE SEARCH
SHEET C**

Application Number

EP 22 15 9146

```
Claim(s) completely searchable:
      11, 12, 27

Claim(s) searched incompletely:
      32

Claim(s) not searched:
      1-10, 13-26, 28-31, 33

Reason for the limitation of the search:

Claims 1, 3, 4, 6, 7, 9-11, 13, 14, 17-20, 21-23, 25-31, 32, 33 have been
drafted as separate independent claims.
In reply to the invitation pursuant to Rule 62a(1) EPC, the applicant has
specified that claim 11 and corresponding method claim 27 and medium
claim 32 should be searched. The applicant has also filed an amended set
of claims, with a request to search amended claims. No amended claim set
can be submitted at this stage of the procedure, hence the request to
search any amended claims cannot be granted. Hence this ESOP refers to
the specified claims in the reply which are part of originally submitted
claims, namely claims 11, 12, 27 and 32.
The applicant's attention is drawn to the fact that the application will
be further prosecuted on the basis of subject-matter for which a search
has been carried out and that the claims should be limited to that
subject-matter at a later stage of the proceedings (Rule 62a(2) EPC).
```

## ANNEX TO THE EUROPEAN SEARCH REPORT
## ON EUROPEAN PATENT APPLICATION NO.

EP 22 15 9146

This annex lists the patent family members relating to the patent documents cited in the above-mentioned European search report.
The members are as contained in the European Patent Office EDP file on
The European Patent Office is in no way liable for these particulars which are merely given for the purpose of information.

02-11-2022

| Patent document cited in search report | | | Publication date | Patent family member(s) | | | Publication date |
|---|---|---|---|---|---|---|---|
| US 2019272887 | A1 | | 05-09-2019 | AU | 2019231261 | A1 | 01-10-2020 |
| | | | | CA | 3093260 | A1 | 12-09-2019 |
| | | | | CN | 112204402 | A | 08-01-2021 |
| | | | | EP | 3762730 | A1 | 13-01-2021 |
| | | | | JP | 2021515233 | A | 17-06-2021 |
| | | | | KR | 20200128710 | A | 16-11-2020 |
| | | | | US | 2019272887 | A1 | 05-09-2019 |
| | | | | WO | 2019173407 | A1 | 12-09-2019 |
| US 2018341754 | A1 | | 29-11-2018 | EP | 3427170 | A1 | 16-01-2019 |
| | | | | US | 2018341754 | A1 | 29-11-2018 |
| | | | | WO | 2018213767 | A1 | 22-11-2018 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017037378 A
- WO 2003054743 A
- JP 2020523010 A
- JP 2020519246 A
- JP 2021035648 A

**Non-patent literature cited in the description**

- **SHO ITO ; AKINOBU SENOO ; SATORU NAGATOISHI ; MASAHITO OHUE ; MASAKI YAMAMOTO ; KOUHEI TSUMOTO ; NAOKI WAKUI.** Structural Basis for the Binding Mechanism of Human Serum Albumin Complexed with Cyclic Peptide Dalbavancin. *J. Med. Chem.,* 13 November 2020, vol. 63 (22), 14045-14053